(19) **Europäisches Patentamt**
**European Patent Office**
**Office européen des brevets**

(11) **EP 3 168 598 A1**

(12) **EUROPEAN PATENT APPLICATION**
published in accordance with Art. 153(4) EPC

(43) Date of publication:
**17.05.2017 Bulletin 2017/20**

(21) Application number: **15819161.9**

(22) Date of filing: **08.07.2015**

(51) Int Cl.:
**G01N 19/00** *(2006.01)*      **G01N 29/04** *(2006.01)*

(86) International application number:
**PCT/JP2015/003431**

(87) International publication number:
**WO 2016/006235 (14.01.2016 Gazette 2016/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **10.07.2014   JP 2014142270**
**26.03.2015   JP 2015064921**

(71) Applicant: **Highfrequency Viscoelasticity Corporation**
**Yokohama-shi, Kanagawa 224-0007 (JP)**

(72) Inventor: **OMATA, Nobuaki**
**Yokohama-shi**
**Kanagawa 224-0007 (JP)**

(74) Representative: **GIE Innovation Competence Group**
**310, avenue Berthelot**
**69372 Lyon Cedex 08 (FR)**

(54) **VISCOELASTIC PROPERTY MEASURING DEVICE AND VISCOELASTIC PROPERTY MEASURING METHOD**

(57)    A viscoelastic characteristic measurement apparatus (1) according to the present invention includes: a sample deforming unit (11) that applies an external force to a measurement sample (S) to impose a cyclic deformation; a first viscoelastic characteristic calculation unit (15) that calculates a low-frequency viscoelastic characteristic of the measurement sample (S) based on stress acting on the measurement sample (S) that has been deformed by the sample deforming unit (11) and distortion of the measurement sample (S); an emission unit (12) that emits an incident sound wave to the measurement sample (S); a receiver (12) that receives a reflected sound wave generated as a result of reflection of the incident sound wave emitted from the emission unit (25) in the measurement sample (S) or a transmitted sound wave generated as a result of transmission of the incident sound wave through the measurement sample (S); and a second viscoelastic characteristic calculation unit (15) that calculates a high-frequency viscoelastic characteristic of the measurement sample (S) at a frequency of the incident sound wave based on the reflected sound wave or the transmitted sound wave received by the receiver (25).

Fig. 1

## Description

## Technical Field

The present invention relates to a viscoelastic characteristic measurement apparatus and a viscoelastic characteristic measurement method.

## Background Art

The quality of rubber products is determined based on viscoelastic characteristics thereof. Performance of a tire, for example, is determined based on a value of the frictional coefficient thereof. Various proposals have been made regarding techniques for measuring the viscoelastic characteristics of these rubber products.

Patent Literature 1 discloses, for example, a technique of measuring frictional characteristics in a viscoelastic body such as a tire. According to Patent Literature 1, a sensor unit emits a sound wave that generates vibrations in a measurement sample as an incident sound wave. Further, the sensor unit receives a reflected sound wave that is generated as a result of reflection of the sound wave in the measurement sample. An operational processing unit calculates a loss tangent in the viscoelastic characteristic of the measurement sample based on the reflected sound wave received by the sensor unit. The operational processing unit calculates the frictional characteristics based on the loss tangent. Patent Literature 2 discloses a technique of measuring dynamic viscoelastic characteristics of a grinding pad by applying periodic vibrations to the grinding pad. Further, Patent Literature 3 discloses a technique of measuring dynamic viscoelastic characteristics of an object by applying rotational vibrations to the object.

## Citation List

## Patent Literature

[Patent Literature 1] Japanese Unexamined Patent Application Publication No. 2007-47130
[Patent Literature 2] Japanese Unexamined Patent Application Publication No. 2004-228265
[Patent Literature 3] Japanese Unexamined Patent Application Publication No. 2010-48722

## Summary of Invention

## Technical Problem

Rubber products such as tires are used in a state in which a large deformation occurs because a large stress is applied to these products. It is known that rubber is a material having material properties which differ between a state in which a large deformation occurs and a state in which a small deformation (e.g., a minor distortion) occurs (Payne effect). Specifically, a large deformation of the rubber material causes destruction of aggregated particles in the material, which causes a reduction in the elastic modulus. Accordingly, experiments on (dynamic measurements on) the rubber material need to be conducted in a condition close to a condition in which the material is actually used.

Frictional characteristics of the rubber material have two factors: adhesive friction and hysteresis friction. Concerning these two factors, it is generally said that a viscoelastic characteristic in a high frequency (hereinafter it will be referred to as a high-frequency viscoelastic characteristic) greatly contributes to the hysteresis friction. As described above, in order to measure the hysteresis friction in a tire or the like, measurements need to be conducted in a condition close to the condition in which the tire is actually used (in a state in which a large deformation occurs). However, Patent Literature 1 does not disclose occurrence of deformation in the measurement sample. When the high-frequency viscoelasticity of the tire is measured using ultrasound waves, since the amplitude of the rubber material generated due to the ultrasonic waves is about one micron, distortion of an actual large deformation cannot be reproduced.

The present invention has been made in order to solve the aforementioned problem and aims to provide a viscoelastic characteristic measurement apparatus and a viscoelastic characteristic measurement method capable of measuring viscoelastic characteristics in a state in which an actual use state of a measurement sample such as a rubber product is appropriately reflected.

## Solution to Problem

A viscoelastic characteristic measurement apparatus according to a first aspect of the present invention includes a sample deforming unit, a first viscoelastic characteristic calculation unit, an emission unit, a receiver, and a second viscoelastic characteristic calculation unit. The sample deforming unit applies an external force to a measurement sample to impose a cyclic deformation. The first viscoelastic characteristic calculation unit calculates a low-frequency viscoelastic characteristic of the measurement sample based on stress acting on the measurement sample that has been deformed by the sample deforming unit and distortion of the measurement sample. The emission unit emits an incident sound wave to the measurement sample that has been deformed by the sample deforming unit. The receiver receives a reflected sound wave generated as a result of reflection of the incident sound wave emitted from the emission unit in the measurement sample or a transmitted sound wave generated as a result of transmission of the incident sound wave through the measurement sample. The second viscoelastic characteristic calculation unit calculates a high-frequency viscoelastic characteristic of the measurement sample at a frequency of the incident sound wave based on the reflected sound wave or the transmitted sound wave received by the receiver.

A viscoelastic characteristic measurement method according to a second aspect of the present invention includes the following steps (a) to (e):

(a): a deformation step for applying an external force to a measurement sample to impose a cyclic deformation;
(b): a first calculation step for calculating a low-frequency viscoelastic characteristic of the measurement sample based on stress acting on the measurement sample that has been deformed and distortion of the measurement sample;
(c): an emission step for emitting an incident sound wave to the measurement sample that has been deformed;
(d): a reception step for receiving a reflected sound wave generated as a result of reflection of the incident sound wave that has been emitted in the measurement sample or a transmitted sound wave generated as a result of transmission of the incident sound wave through the measurement sample; and
(e): a second calculation step for calculating a high-frequency viscoelastic characteristic of the measurement sample at a frequency of the incident sound wave based on the reflected sound wave or the transmitted sound wave that has been received.

**Advantageous Effects of Invention**

According to the present invention, it is possible to provide a viscoelastic characteristic measurement apparatus and a viscoelastic characteristic measurement method capable of measuring viscoelastic characteristics in a state in which an actual use state of a measurement sample such as a rubber product is appropriately reflected.

**Brief Description of Drawings**

Fig. 1 is a block diagram showing a configuration example of a viscoelastic characteristic measurement apparatus according to a first embodiment;
Fig. 2 is a block diagram showing a configuration example of a sensor according to the first embodiment;
Fig. 3 is a block diagram showing a configuration example of a processing unit according to the first embodiment;
Fig. 4A is a diagram for describing a method of calculating a high-frequency viscoelastic characteristic according to the first embodiment;
Fig. 4B is a diagram for describing the method of calculating the high-frequency viscoelastic characteristic according to the first embodiment;
Fig. 5A is a flowchart showing one example of processing of the viscoelastic characteristic measurement apparatus according to the first embodiment;
Fig. 5B is a flowchart showing one example of processing of the viscoelastic characteristic measurement apparatus according to the first embodiment;
Fig. 6 is a block diagram showing a configuration example of a viscoelastic characteristic measurement apparatus according to a second embodiment;
Fig. 7A is a diagram for describing a method of calculating a high-frequency viscoelastic characteristic according to the second embodiment;
Fig. 7B is a diagram for describing the method of calculating the high-frequency viscoelastic characteristic according to the second embodiment;
Fig. 7C is a diagram for describing the method of calculating the high-frequency viscoelastic characteristic according to the second embodiment;
Fig. 8A is a flowchart showing one example of processing of the viscoelastic characteristic measurement apparatus according to the second embodiment;

Fig. 8B is a flowchart showing one example of processing of the viscoelastic characteristic measurement apparatus according to the second embodiment;

Fig. 9 is a block diagram showing a configuration example of a viscoelastic characteristic measurement apparatus according to a third embodiment;

Fig. 10A is a diagram showing a measurement example of a high-frequency viscoelastic characteristic when a bending force is applied to a measurement sample;

Fig. 10B is a diagram showing a measurement example of the high-frequency viscoelastic characteristic when the bending force is applied to the measurement sample;

Fig. 10C is a diagram showing a measurement example of the high-frequency viscoelastic characteristic when the bending force is applied to the measurement sample;

Fig. 11 is a diagram showing a measurement example of a low-frequency viscoelastic characteristic when the measurement sample is rotated about a rotation axis according to a predetermined frequency and an electric field is applied to the measurement sample;

Fig. 12 is a block diagram showing a configuration example of a viscoelastic characteristic measurement apparatus according to a fourth embodiment;

Fig. 13A is a diagram for describing a method of calculating a high-frequency viscoelastic characteristic according to the fourth embodiment;

Fig. 13B is a diagram for describing the method of calculating the high-frequency viscoelastic characteristic according to the fourth embodiment;

Fig. 13C is a diagram for describing the method of calculating the high-frequency viscoelastic characteristic according to the fourth embodiment;

Fig. 14 is a block diagram showing a configuration example of the viscoelastic characteristic measurement apparatus according to the fourth embodiment;

Fig. 15 is a graph showing one example of a relation between distortion and stress in the measurement sample having viscoelastic characteristics;

Fig. 16 is a graph showing one example of a relation between a frequency and viscoelasticity in the measurement sample having the viscoelastic characteristics; and

Fig. 17 is a block diagram showing a partial configuration example of a viscoelastic characteristic measurement apparatus according to a fifth embodiment.

**Description of Embodiments**

[First Embodiment]

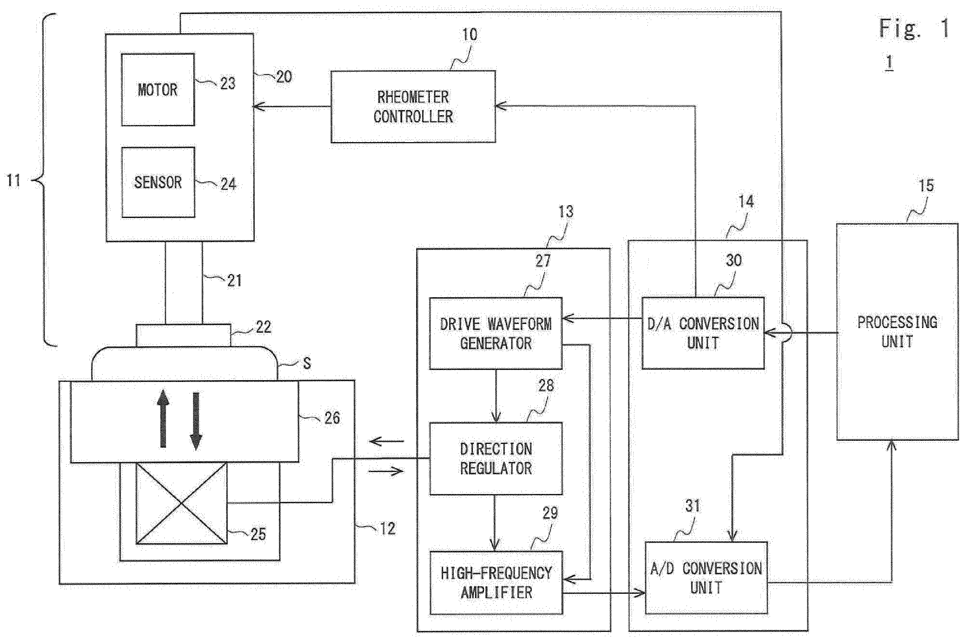

Hereinafter, with reference to the drawings, a first embodiment of the present invention will be described. Fig. 1 is a block diagram showing a configuration example of a viscoelastic characteristic measurement apparatus 1 according to the first embodiment. The viscoelastic characteristic measurement apparatus 1 includes a rheometer controller 10, a rheometer unit 11, a sound wave emission unit 12, a sound wave signal generation unit 13, a conversion unit 14, and a processing unit 15. In the following description, a measurement sample whose viscoelastic characteristic is to be measured will be described as a measurement sample S. The measurement sample S may either be a solid sample or a liquid sample. Further, the viscoelastic characteristic includes at least one value of a loss tangent $\tan\delta$, a storage elastic modulus E', and a loss elastic modulus E" that will be described later.

First, the rheometer controller 10 will be described. The rheometer controller 10 acquires, from the processing unit 15, a control signal that instructs an operation of the rheometer unit 11. The rheometer controller 10 controls the rheometer unit 11 according to the control signal to cause the rheometer unit 11 to apply a predetermined distortion to the measurement sample S. Specifically, the rheometer controller 10 outputs a drive instruction to a motor 23 of a drive unit 20.

The rheometer controller 10 is able to control the rheometer unit 11 to cause the rheometer unit 11 to rotate about an axis 21 (rotation jig). The part of the measurement sample S on the side of the axis 21 moves in accordance with the rotation of the rheometer unit 11 and the part of the measurement sample S on the side of the sound wave emission unit 12 (part of the measurement sample S on the side opposite to the side of the axis 21) is fixed and does not move. Therefore, deformation of torsion (shear) is generated in the measurement sample S. Further, the rheometer controller 10 may make the axis 21 vibrate in the length direction (perpendicular direction in Fig. 1: hereinafter this direction will be referred to as a vertical direction) to make the measurement sample S vibrate in the vertical direction. The rheometer controller 10 may control, for example, rotation or vibrations of the axis 21 and generate sine wave vibrations in the measurement sample S.

In the aforementioned case, the rheometer controller 10 is able to adjust the width and the cycle of the rotation of the axis 21 or the amplitude and the frequency of the vibrations of the axis 21. The rheometer controller 10 performs control so that the measurement sample S is rotated or vibrated at a low frequency (e.g., about several Hz to 100 Hz).

Alternatively, the rheometer controller 10 may perform control so that the motor 23 applies stress to the measurement sample S via the axis 21 in the vertical direction at a predetermined cycle to compress the measurement sample S. In another alternative, the rheometer controller 10 may perform control so that the motor 23 pulls up the measurement sample S via the axis 21 in the vertical direction at a predetermined cycle to stretch the measurement sample S. In this case, the rheometer controller 10 is able to adjust the magnitude and the cycle of the pressure or tension. In one more alternative, the rheometer controller 10 may apply a bending force to the measurement sample S from a width direction of the axis 21 (horizontal direction in Fig. 1: hereinafter this direction will be referred to as a lateral direction) at a predetermined cycle. In this way, the rheometer controller 10 controls the rheometer unit to apply an external force to the measurement sample S, to thereby impose a cyclic deformation.

The operations of the rheometer controller 10 stated above may be executed in combination with each other. The rheometer controller 10 can be formed of, for example, a circuit such as a memory or another integrated circuit (IC).

The rheometer unit 11 is a sample deforming unit that applies an external force to the measurement sample S in accordance with the control of the rheometer controller 10 to deform the measurement sample S. The deformation of the measurement sample S generated in accordance with the operation of the rheometer unit 11 has been stated above. The measurement sample S is sandwiched between the rheometer unit 11 and the sound wave emission unit 12.

The rheometer unit 11 specifically includes the drive unit 20, the axis 21, and an annular plate 22. The drive unit 20 applies the external force to the measurement sample S via the axis 21. Specifically, the drive unit 20 includes the motor 23 and a sensor 24. The motor 23 drives the axis 21 in accordance with the drive instruction from the rheometer controller 10. The axis 21 is connected to the annular plate 22 and the annular plate 22 is moved in accordance with the movement of the axis 21. Since the annular plate 22 is fixed to one surface of the measurement sample S, the measurement sample S is deformed according to the external force applied from the drive unit 20.

Further, the annular plate 22 is provided with a probe capable of acquiring the temperature of the measurement sample S. The information on the temperature of the measurement sample S acquired by the probe is output to the sensor 24.

The sensor 24 measures a measurement amount regarding the state of the measurement sample S and outputs the measurement amount to the conversion unit 14. The measurement amount that has been output to the conversion unit 14 is converted by the conversion unit 14 and the resulting value is output to the processing unit 15.

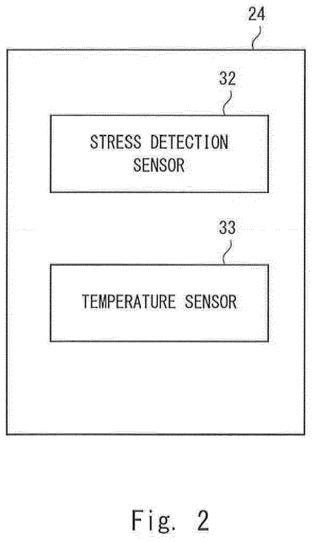

Fig. 2 is a block diagram showing a configuration example of the sensor 24. Specifically, the sensor 24 includes a stress detection sensor 32 and a temperature detection sensor 33. In the following description, each component of the sensor 24 will be described.

The stress detection sensor 32 detects the stress acting on the measurement sample S based on the drive instruction output from the rheometer controller 10 and outputs data of the stress that has been detected to the conversion unit 14. When the drive instruction from the rheometer controller 10 is to apply vibrations in the vertical direction based on a sine wave to the measurement sample S, for example, the stress detection sensor 32 detects the data of the stress of the sine wave and outputs the detected data to the conversion unit 14. When a shearing force is applied to the measurement sample S, the stress detection sensor 32 may detect the stress acting on the measurement sample S by detecting torque that has been generated.

The temperature detection sensor 33 acquires the temperature of the measurement sample S from the probe provided in the annular plate 22. The temperature detection sensor 33 outputs the data of the temperature that has been acquired to the conversion unit 14.

Referring back to Fig. 1, the descriptions will be continued. The sound wave emission unit 12 contacts the measurement sample S, emits a high-frequency incident sound wave generated by the sound wave signal generation unit 13 to the measurement sample S, and acquires the reflected sound wave (measurement amount) generated as a result of reflection of the incident sound wave in the measurement sample S. Specifically, the sound wave emission unit 12 includes a transducer 25 and a delay member 26. In the following description, each component will be described.

The transducer 25 is formed to include, for example, piezoelectric elements. The transducer 25 is provided in such a way that it contacts the delay member 26. The transducer 25 is connected to a direction regulator 28 that will be described later and converts, when receiving an electric signal from the direction regulator 28, this electric signal into a sound wave. The transducer 25 emits (outputs) the sound wave after the conversion to the delay member 26. The delay member 26 delays the sound wave that has been emitted and emits the delayed wave to the measurement sample S. Further, upon receiving the reflected sound wave (sound wave generated as a result of the reflection of the incident sound wave in the measurement sample S) emitted from the measurement sample S via the delay member 26, the transducer 25 converts the reflected sound wave into an electric signal. The transducer 25 outputs the electric signal after the conversion to the direction regulator 28.

The delay member 26 has one surface that is in close contact with the transducer 25 and another surface opposed to the surface that contacts the measurement sample S. According to such a configuration, the delay member 26 is able to propagate the incident sound wave emitted from the transducer 25 to the measurement sample S and to propagate the reflected sound wave that is generated as a result of the reflection of the incident sound wave in the measurement sample S to the transducer 25. The delay member 26 serves to delay the arrival time of the sound wave. More specifically, by increasing the propagation length of the delay member 26, a time from the emission of the incident sound wave by the transducer 25 until the reception of the reflected sound wave by the transducer 25 can be increased. It is therefore possible to prevent the transducer 25 from receiving the reflected sound wave while it is emitting the incident sound wave.

Next, the sound wave signal generation unit 13 will be described. The sound wave signal generation unit 13 generates the electric signal of the incident sound wave and outputs the electric signal to the sound wave emission unit 12 to calculate the high-frequency viscoelastic characteristic. Further, the sound wave signal generation unit 13 receives the electric signal of the reflected sound wave acquired by the sound wave emission unit 12 and outputs the electric signal that has been received to the conversion unit 14. The sound wave signal generation unit 13 specifically includes a drive waveform generator 27, the direction regulator 28, and a high-frequency amplifier 29. In the following description, these components will be described.

The drive waveform generator 27 generates the electric signal (drive waveform) in accordance with a signal for instructing emission of the sound wave output from the processing unit 15 and outputs the electric signal that has been generated to the direction regulator 28. This electric signal is a signal to generate the sound wave to be emitted to the measurement sample S. Specifically, the sound wave to be emitted to the measurement sample S may be a pulse-like sound wave or a sound wave that includes a predetermined frequency component. Further, when the drive waveform generator 27 generates the aforementioned electric signal and outputs the generated electric signal, the drive waveform generator 27 outputs a trigger signal indicting the timing when the electric signal that has been generated is to be output to the high-frequency amplifier 29.

The direction regulator 28 is connected to the drive waveform generator 27, the high-frequency amplifier 29, and the transducer 25. The direction regulator 28 outputs the electric signal received from the drive waveform generator 27 to the transducer 25 and outputs the electric signal supplied from the transducer 25 to the high-frequency amplifier 29. The direction regulator 28 adjusts the signal transmission direction in such a way that the electric signal output from the drive waveform generator 27 is not output to the high-frequency amplifier 29.

The high-frequency amplifier 29 receives the electric signal supplied from the direction regulator 28. This electric signal is output from the transducer 25. The high-frequency amplifier 29 amplifies the high-frequency components in the electric signal that has been supplied at a predetermined amplification rate. Then the high-frequency amplifier 29 outputs the electric signal after the amplification to the conversion unit 14. The high-frequency components (e.g., 1 MHz to 100 MHz) in the electric signal amplified by the high-frequency amplifier 29 include the measurement amount that is required to calculate the high-frequency viscoelastic characteristic. After the high-frequency amplifier 29 receives the trigger signal from the drive waveform generator 27, the high-frequency amplifier 29 starts receiving the electric signal supplied from the transducer 25. According to the above processing, the high-frequency amplifier 29 does not perform operations for a period during which the high-frequency viscoelastic characteristic of the measurement sample S is not measured. It is therefore possible to suppress undesired operations of the high-frequency amplifier 29.

In this way, the sound wave emission unit 12 and the sound wave signal generation unit 13 measure the measurement amount regarding the high-frequency viscoelastic characteristic of the measurement sample S. Further, the sound wave emission unit 12 serves as an emission unit that outputs the incident sound wave to the measurement sample S and also serves as a receiver that receives the reflected sound wave that is generated as a result of the reflection of the incident sound wave in the measurement sample S. Such a configuration is often used in non-destructive inspections (ultrasound waves).

Next, the conversion unit 14 will be described. The conversion unit 14 converts formats of signals communicated between the processing unit 15 and the rheometer controller 10, the drive unit 20, and the sound wave signal generation unit 13. More specifically, the conversion unit 14 includes a D/A conversion unit 30 and an A/D conversion unit 31. The D/A conversion unit 30 converts a digital control signal output from the processing unit 15 into an analog signal and outputs the control signal after the conversion to the rheometer controller 10. Further, the D/A conversion unit 30 converts a digital emission instruction signal output from the processing unit 15 into an analog signal and outputs the emission instruction signal after the conversion to the drive waveform generator 27.

Further, the A/D conversion unit 31 converts the electric signal output from the high-frequency amplifier 29 from an analog signal into a digital signal and outputs the electric signal after the conversion to the processing unit 15. Further, the A/D conversion unit 31 converts the electric signal output from the sensor 24 from an analog signal into a digital signal and outputs the electric signal after the conversion to the processing unit 15. The D/A conversion unit 30 is composed of a D/A conversion circuit (converter) and the A/D conversion unit 31 is composed of an A/D conversion circuit (converter). In the following description, the descriptions of the A/D conversion of the signal to be output to the processing unit 15 and the D/A conversion of the signal output from the processing unit 15 by the conversion unit 14 described above will be omitted as appropriate.

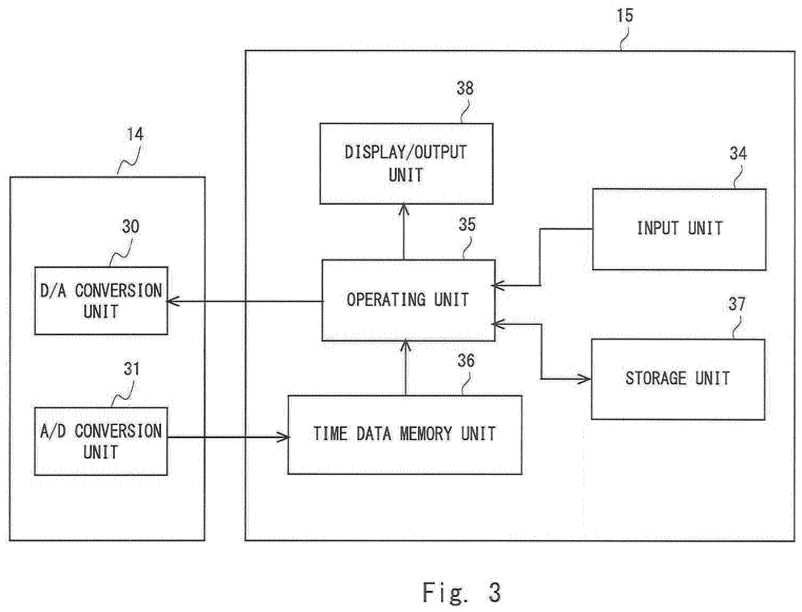

Next, the processing unit 15 (a first viscoelastic characteristic calculation unit and a second viscoelastic characteristic calculation unit) will be described. Fig. 3 is a block diagram showing a configuration example of the processing unit 15. The processing unit 15 calculates the high-frequency viscoelastic characteristic of the measurement sample S using the measurement amounts measured by the sound wave emission unit 12 and the sound wave signal generation unit 13. More specifically, the processing unit 15 includes an input unit 34, an operating unit 35, a time data memory unit 36, a storage unit 37, and a display/output unit 38. The processing unit 15 is composed of, for example, a computer (in particular, a personal computer). Each component of the system in the processing unit 15 as a functional block that performs various processing may be formed of a circuit such as a memory or another IC in hardware and may be implemented by a program or the like loaded to the memory in advance. In the following description, each component will be described.

A deformation instruction indicating the type and parameters of the deformation of the measurement sample S executed by the rheometer unit 11 is input to the input unit 34 by a measurer. The measurer is able to input, for example, an instruction to rotate the measurement sample S (that is, to rotate the axis 21) and parameters such as the width and the cycle of the rotation to the input unit 34. The same is applicable to a case in which the measurement sample S is stretched, compressed, or vibrated. In this way, the measurer determines a predetermined distortion to be applied to the measurement sample S.

Further, the measurer may input an emission instruction to cause the sound wave signal generation unit 13 to emit the sound wave to the input unit 34. The input unit 34 is composed of, for example, buttons.

When the deformation instruction indicating the type and the parameters of the deformation of the measurement sample S executed by the rheometer unit 11 is output from the input unit 34, the operating unit 35 outputs the control signal to the rheometer controller 10. The operating unit 35 controls the operation of the rheometer unit 11 with this control signal. Further, the operating unit 35 stores data of the distortion of the measurement sample S generated in accordance with the deformation instruction in the storage unit 37.

The sensor 24 outputs data of the measurement amount (data of the stress acting on the measurement sample S and the temperature of the measurement sample S) to the time data memory unit 36. The operating unit 35 reads out the data stored in the time data memory unit 36 and the data of the distortion of the measurement sample S stored in the storage unit 37 to calculate a viscoelastic characteristic in a low frequency (hereinafter this characteristic will be referred to as a low-frequency viscoelastic characteristic) of the measurement sample S.

Further, the operating unit 35 outputs, upon receiving the instruction to emit the sound wave from the input unit 34, the emission instruction signal to the D/A conversion unit 30. The D/A conversion unit 30 converts the emission instruction signal from a digital signal into an analog signal and outputs the emission instruction signal after the conversion to the drive waveform generator 27. In this way, the operating unit 35 generates the sound wave to be emitted to the measurement sample S. These signals output from the operating unit 35 are, as described above, digital signals. The drive waveform generator 27 generates the electric signal to cause generation of the sound wave to be emitted to the measurement sample S in accordance with the emission instruction signal to output the electric signal to the direction regulator 28. In this way, the operating unit 35 starts measuring the high-frequency viscoelastic characteristic in the measurement sample S upon receiving the instruction from the input unit 34 to emit the sound wave.

When the time waveform data of the reflected sound wave is stored in the time data memory unit 36, the operating unit 35 reads out the data. The operating unit 35 performs waveform analysis processing in a frequency region such as, for example, Fast Fourier Transformation (FFT) processing to acquire the amplitude value and the phase at the frequency to be detected. The number of frequencies to be detected may either be one or plural. Next, the operating unit 35 reads out a reference value stored in the storage unit 37 and calculates the high-frequency viscoelastic characteristic of the measurement sample S based on the reference value and the amplitude value and the phase of the reflected sound wave stored in the time data memory unit 36 at the frequency to be detected.

The data output from the sensor 24 is stored in the time data memory unit 36 at a predetermined cycle. The data to be stored is data of the stress acting on the measurement sample S and the temperature of the measurement sample S.

Further, the time data memory unit 36 stores a time waveform of the electric signal of the reflected sound wave supplied from the high-frequency amplifier 29 at a predetermined cycle. The time data memory unit 36 is able to change the cycle at which data is stored based on the control of the operating unit 35.

The storage unit 37 stores data of the distortion of the measurement sample S and stores the reference value that is necessary to calculate the high-frequency viscoelastic characteristic of the measurement sample S in advance. This reference value is data of the amplitude value and the phase at the frequency at which the high-frequency viscoelastic characteristic is measured. The details of the reference value will be described later. The operating unit 35 reads out the reference value stored in the storage unit 37.

The display/output unit 38 displays or output the low-frequency viscoelastic characteristic and the viscoelastic characteristic in the high frequency of the measurement sample S calculated in the operating unit 35 to the measurer. The display/output unit 38 is, for example, a display.

<Method of calculating Low-Frequency Viscoelastic Characteristic>

Next, a case in which the viscoelastic characteristic measurement apparatus 1 calculates the low-frequency viscoelastic characteristic of the measurement sample S will be briefly described. The operating unit 35 reads data of the stress acting on the measurement sample S from the time data memory unit 36 and reads data of the distortion of the measurement sample S from the storage unit 37. The operating unit 35 then compares the amplitude of the stress acting on the measurement sample S with the amplitude of the distortion of the measurement sample S to calculate the amplitude ratio of them. This amplitude ratio is the storage elastic modulus E' in the low frequency. Further, the operating unit 35 compares the phase of the stress acting on the measurement sample S with the phase of the distortion of the measurement sample S to calculate the phase difference between them. The operating unit 35 calculates the loss tangent tanδ in the low frequency using this phase difference. When the loss elastic modulus is denoted by E", the following relation is established among the loss tangent tanδ, the storage elastic modulus E', and the loss elastic modulus E".

$$\tan\delta = E''/E' \cdots (1)$$

Therefore, by calculating the loss tangent tanδ and the storage elastic modulus E', the loss elastic modulus E" can be calculated as well. In this way, the operating unit 35 is able to calculate a dynamic low-frequency viscoelastic characteristic (see, for example, Patent Literature 2).
Further, the operating unit 35 is able to calculate the temperature dependency of the low-frequency viscoelastic characteristic using the data of the temperature of the measurement sample S read out from the time data memory unit 36.

<Method of calculating High-Frequency Viscoelastic Characteristic (Surface Reflection Method)>

Further, a case in which the viscoelastic characteristic measurement apparatus 1 calculates the high-frequency viscoelastic characteristic of the measurement sample S will be described. In this calculation method, a surface reflection method is used (see, for example, Patent Literature 1). In this case, the transducer 25 emits the incident sound wave to the measurement sample S. The incident sound wave that has been emitted is reflected in the surface of the measurement sample S and the reflected sound wave is generated. The processing unit 15 measures the high-frequency viscoelastic characteristic based on the reflected sound wave.

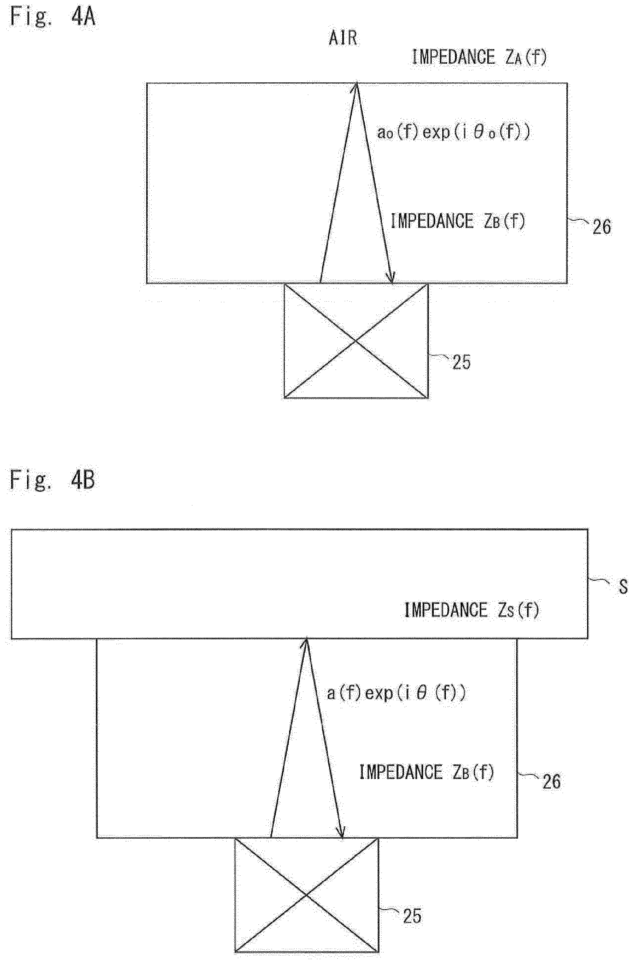

Figs. 4A and 4B are diagrams for describing the method of calculating the high-frequency viscoelastic characteristic using this surface reflection method. Fig. 4A is a diagram showing the reflection status of the incident sound wave when the reference value is acquired and Fig. 4B is a diagram showing the reflection status of the incident sound wave when the high-frequency viscoelastic characteristic of the measurement sample S is calculated. In the following description, an acoustic impedance that indicates the propagation characteristic of the incident sound wave emitted from the transducer 25 of the sound wave emission unit 12 is used.
Referring first to Fig. 4A, the reference value will be described. The reference value is a phase and an amplitude value at the frequency of the measurement sample when the surface of the delay member 26 opposite to the surface that the transducer 25 contacts does not contact the measurement sample S. In this case, the incident sound wave is reflected on the interface between an end of the delay member 26 and the air. In the following description, the frequency of the incident sound wave and the reflected sound wave is denoted by f and the acoustic impedance of the delay member 26 is denoted by $Z_B(f)$, which is a function of the frequency f. In a similar way, the acoustic impedance in the air is denoted by $Z_A(f)$, which is a function of the frequency f. The acoustic impedances $Z_B(f)$ and $Z_A(f)$ are complex values.
The reflectance $R_{BA}(f)$ of the incident sound wave in the interface between the delay member 26 and the air is as follows.

$$R_{BA}(f) = (Z_A(f) - Z_B(f))/(Z_A(f) + Z_B(f)) \cdots (2)$$

In this case, at a desired frequency f, $Z_A(f)$ is much smaller than $Z_B(f)$. Therefore, from Expression (2), the reflectance $R_{AB}(f)$ becomes -1. That is, the entire incident sound wave is reflected on the interface between the delay member 26 and the air.
In the following description, the expression of the reflected sound wave that is incident on the transducer 25 is expressed by $a_0(f)\exp(i\theta_0(f))$. The symbol i denotes an imaginary unit, the symbol $a_0(f)$ denotes an amplitude value of a real number at the target frequency, and the symbol $\theta_0(f)$ is a real number equal to or larger than 0 and indicates the phase at each frequency. The expression of the incident sound wave emitted from the transducer 25 to the measurement sample S is expressed as follows.

$$a_0(f)\exp(i\theta_0(f))\times R_{AB}(f)=-a_0(f)\exp(i\theta_0(f))\cdots(3)$$

It can therefore be regarded that the incident sound wave shown in Expression (3) is emitted to the measurement sample S. The storage unit 37 stores the amplitude $a_0(f)$ and the phase $\theta_0(f)$ in Expression (3) as a reference value in advance. This reference value is measured and obtained in advance.

With reference next to Fig. 4B, a case in which the high-frequency viscoelastic characteristic of the measurement sample S is calculated will be described. When the high-frequency viscoelastic characteristic of the measurement sample S is calculated, in a state in which the delay member 26 is in close contact with the measurement sample S, the incident sound wave the same as that shown in Fig. 4A is emitted from the transducer 25 according to the output of the electric signal from the drive waveform generator 27. The transducer 25 receives the reflected sound wave reflected on the interface between the delay member 26 and the measurement sample S and the high-frequency amplifier 29 amplifies the high-frequency components in the electric signal of the reflected sound wave. The operating unit 35 compares the reflected sound wave with the reference value stored in the storage unit 37 to calculate the loss tangent of the measurement sample S.

When the acoustic impedance of the measurement sample S, which is a function of the frequency f, is denoted by $Z_S(f)$, a reflectance $R_{BS}(f)$ of the incident sound wave in the interface between the delay member 26 and the measurement sample S is as follows.

$$R_{BS}(f)=(Z_S(f)-Z_B(f))/(Z_S(f)+Z_B(f))\cdots(4)$$

From Expression (4), $Z_S(f)$ can be expressed as follows.

$$Z_S(f)=Z_B(f)\times(1+R_{BS}(f))/(1-R_{BS}(f))\cdots(5)$$

In the following description, the expression of the reflected sound wave incident on the transducer 25 is expressed by $a(f)\exp(i\theta(f))$. The symbol i indicates an imaginary unit, the symbol $a(f)$ indicates an amplitude value of a real number at the target frequency, and the symbol $\theta(f)$ is a real number equal to or larger than 0 and indicates the phase at each frequency. Using the reference value in Expression (2), the expression of the reflected sound wave is expressed by the following expression.

$$a(f)\exp(i\theta(f))=-a_0(f)\exp(i\theta_0(f))\times R_{BS}(f)\cdots(6)$$

From Expression (5), the reflectance $R_{BS}(f)$ of the incident sound wave is expressed by the following expression.

$$R_{BS}(f)=-(a(f)/a_0(f))\times\exp(i(\theta(f)-\theta_0(f))\cdots(7)$$

When Expression (6) is substituted into Expression (4), $Z_S(f)$ can be obtained as follows.

$$Z_S(f)=Z_B(f)\times(1-(a(f)/a_0(f))\times\exp(i(\theta(f)-\theta_0(f)))/(1+(a(f)/a_0(f))\times\exp(i(\theta(f)-\theta_0(f)))\cdots(8)$$

The storage elastic modulus and the loss elastic modulus of the measurement sample S, which is a function of the frequency f, are respectively denoted by E'(f) and E"(f). In this case, the following relation is established between E'(f) and E"(f) and the acoustic impedance $Z_S(f)$ and the density $\rho_T$ of the measurement sample S.

$$E'+iE''(f)=Z_S(f)^2/\rho_T\cdots(9)$$

By substituting Expression (8) into Expression (9) and separating the real number component and the imaginary number component, a loss tangent $\tan\delta(f)$ is calculated as follows.

$$\tan\delta(f)=E''/E'=\{4\times(a(f)/a_0(f))\times(1-(a(f)/a_0(f))^2)\times\sin(\theta(f)-\theta_0(f))\}/\{(1-(a(f)/a_0(f))^2)^2-4\times(a(f)/a_0(f))^2\times\sin^2(\theta(f)-\theta_0(f))\}\cdots(10)$$

The storage elastic modulus E'(f) and the loss elastic modulus E"(f) are respectively calculated as follows.

$$E'(f)=Re[Z_S(f)^2/\rho_T]=(Z_B(f)^2/\rho_T)\times\{(1-(a(f)/a_0(f))^2)^2-4(a(f)/a_0(f))^2\times\sin^2(\theta(f)-\theta_0(f))\}/\{1+2(a(f)/a_0(f))\cos(\theta(f)-\theta_0(f))+(a(f)/a_0(f))^2\}^2\cdots(11)$$

$$E''(f)=Im[Z_S(f)^2/\rho_T]=(Z_B(f)^2/\rho_T)\times\{4(a(f)/a_0(f))\times(1-(a(f)/a_0(f))^2)\sin(\theta(f)-\theta_0(f))\}/\{1+2(a(f)/a_0(f))\cos(\theta(f)-\theta_0(f))+(a(f)/a_0(f))^2\}^2\cdots(12)$$

The symbol $Re[Z_S(f)^2/\rho_T]$ indicates the real number component of $Z_S(f)^2/\rho_T$ and the symbol $Im[Z_S(f)^2/\rho_T]$ indicates the imaginary number component of $Z_S(f)^2/\rho_T$.

As shown in Expressions (10) to (12), the storage elastic modulus E'(f), the loss elastic modulus E"(f), and the loss tangent $\tan\delta(f)$ are all defined by $\{a(f)/a_0(f)\}$ and $\{\theta(f)-\theta_0(f)\}$ based on $a_0(f)$ and $\theta_0(f)$. The operating unit 35 sets the amplitude $a_0(f)$ and the phase characteristic $\theta_0(f)$ as a reference value and compares the data of the electric signal of the reflected sound wave acquired when the measurement sample S is measured with the reference value. According to this processing, it is possible to measure the high-frequency viscoelastic characteristic of the measurement sample S. Further, as stated above, the high-frequency viscoelastic characteristic of the measurement sample S depends on the frequency. Accordingly, the operating unit 35 may calculate the high-frequency viscoelastic characteristic for each of the plurality of frequency components. Further, when it is required to calculate the loss tangent at a high frequency, ultrasound waves may be supplied from the transducer 25 as the incident sound wave.

Further, the operating unit 35 is able to calculate the temperature dependency of the high-frequency viscoelastic characteristic using data of the temperature of the measurement sample S read out from the time data memory unit 36 as well.

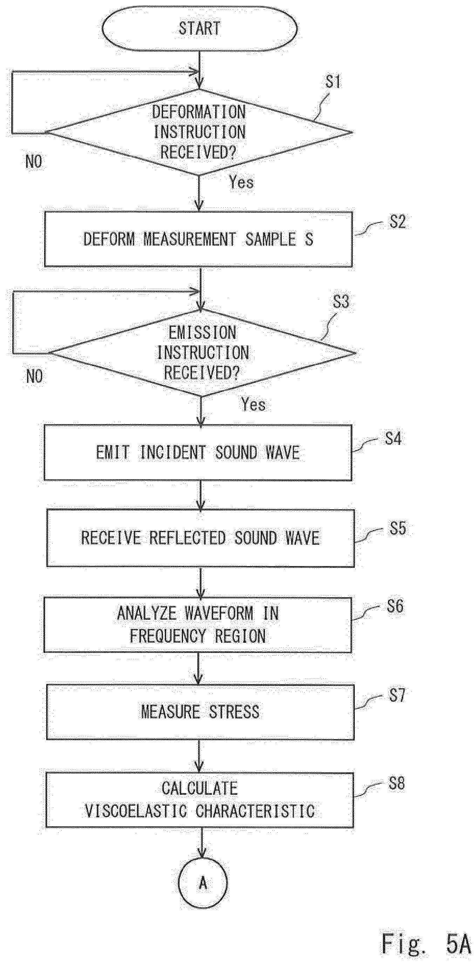

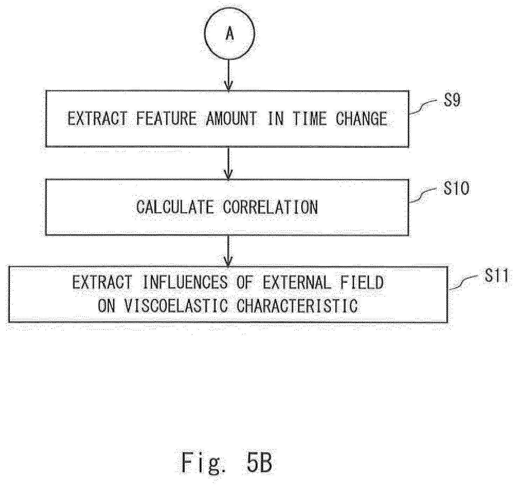

Figs. 5A and 5B are flowcharts showing one example of processing of the viscoelastic characteristic measurement apparatus 1. In the following description, with reference to Figs. 5A and 5B, the whole processing in the viscoelastic characteristic measurement apparatus 1 will be described.

First, the operating unit 35 determines whether it has received the instruction to deform the measurement sample S from the input unit 34 (Step S1). When the operating unit 35 has not received the deformation instruction (No in Step S1), the operating unit 35 does not output the control signal to the rheometer controller 10 and performs the determination processing in Step S1 again. The details of the deformation instruction have been described above.

When the operating unit 35 has received the instruction to deform the measurement sample S (Yes in Step S1), the operating unit 35 outputs the control signal to the rheometer controller 10. The rheometer controller 10 outputs the drive instruction to the motor 23 of the drive unit 20 according to the control signal. The motor 23 is driven according to this drive instruction and the measurement sample S is deformed (Step S2). Further, the operating unit 35 stores data of the distortion of the measurement sample S in the storage unit 37.

Next, the operating unit 35 determines whether it has received the instruction to emit the sound wave from the input unit 34 (Step S3). When it has not received the emission instruction (No in Step S3), the operating unit 35 does not allow generation of the sound wave to be emitted to the measurement sample S and performs the determination processing of Step S3 again. The details of the emission instruction have been stated above.

When the operation unit 35 has received the instruction to emit the sound wave (Yes in Step S3), the operating unit 35 outputs the signal for instructing emission of the sound wave to the drive waveform generator 27. The drive waveform generator 27 generates, in accordance with the emission instruction signal, the electric signal to generate the incident sound wave to be emitted to the measurement sample S to output the electric signal to the direction regulator 28. The transducer 25 converts the electric signal that has been supplied into the incident sound wave and emits the incident sound wave to the measurement sample S via the delay member 26 (Step S4).

Upon receiving the reflected sound wave from the measurement sample S via the delay member 26, the transducer 25 converts the reflected sound wave into the electric signal and outputs the electric signal after the conversion to the direction regulator 28 (Step S5). The direction regulator 28 outputs the electric signal to the high-frequency amplifier 29. The high-frequency amplifier 29 amplifies the high-frequency components included in the electric signal that has been supplied and outputs the electric signal that has been amplified to the time data memory unit 36.

The operating unit 35 reads out the data stored in the time data memory unit 36, performs waveform analysis processing in a frequency region, and acquires the amplitude value and the phase in the frequency to be detected (Step S6). Further, the stress detection sensor 32 detects the stress acting on the measurement sample S (Step S7).

The operating unit 35 calculates the low-frequency viscoelastic characteristic of the measurement sample S based on the data of the stress acting on the measurement sample S and the data of the distortion of the measurement sample S stored in the storage unit 37. Further, the operating unit 35 reads out the reference value stored in the storage unit 37. The operating unit 35 calculates the high-frequency viscoelastic characteristic of the measurement sample S based on the reference value and the amplitude value and the phase at the frequency to be detected of the reflected sound wave stored in the time data memory unit 36 (Step S8). The details of the calculation method have been stated above.

The operating unit 35 calculates and obtain the low-frequency viscoelastic characteristic for a predetermined period of time and extracts the feature amount of the viscoelastic characteristics in the time change (Step S9). The feature amount may be at least one value of the storage elastic modulus E'(f), the loss elastic modulus E"(f), and the loss tangent $\tan\delta$(f) or may be a ratio of two values selected from the storage elastic modulus E'(f), the loss elastic modulus E"(f), and the loss tangent $\tan\delta$(f). Further, the operating unit 35 may perform a differential calculation to detect the peak of the time change of the viscoelastic characteristics as the feature amount or may detect the feature amount that characterizes the degree of increasing/decreasing tendency of the viscoelastic characteristics. As stated above, the operating unit 35 is able to calculate the feature amount using the method of calculating the feature amount that is generally known. Further, the feature amount may use either one of the value of the low-frequency viscoelastic characteristic and the value of the high-frequency viscoelastic characteristic or both the values of the low-frequency viscoelastic characteristic and the high-frequency viscoelastic characteristic. The operating unit 35 is able to determine whether the measurement sample S has the optimal physical property in the application of the measurement sample S by calculating the appropriate feature amount among various feature amounts.

When the measurement sample S changes in a chemical process (the measurement sample S undergoes a chemical change), the spectroscopy of the viscoelastic characteristic of the measurement sample S may change with time. Consider a case in which the measurement sample S is, for example, dough. In this case, when the measurement sample S is kneaded while it is being deformed, the viscoelastic characteristic is shifted with time. Based on this viscoelastic characteristic that is shifted with time, the value of the viscoelastic characteristic optimal for the dough can be determined. Specifically, a plurality of specimens of dough obtained by deforming the measurement samples S made of the same material for different periods of time are generated. By baking these plurality of specimens, the specimen that will become bread having the optimal hardness after the baking is specified. The viscoelastic characteristic data of this specimen becomes the value of the viscoelastic characteristic optimal for the dough. When the measurement sample S is other than dough, the value of the optimal viscoelastic characteristic can be determined in accordance with the characteristic of the measurement sample S.

It is possible to determine the feature amount indicating whether the measurement sample S has the viscoelastic characteristic optimal for the application based on values of the optimal viscoelastic characteristic of the measurement sample S thus acquired (the storage elastic modulus E'(f), the loss elastic modulus E"(f), and the loss tangent $\tan\delta$(f)). When the feature amount of the measurement sample S acquired in Step S9 is in a predetermined range, the measurer determines, for example, that the measurement sample S has the optimal viscoelastic characteristic and when the feature amount of the measurement sample S is outside the predetermined range, the measurer can determine that the measurement sample S does not have the optimal viscoelastic characteristic.

Further, the operating unit 35 calculates the degree of correlation between the high-frequency viscoelastic characteristic and the low-frequency viscoelastic characteristic using the high-frequency viscoelastic characteristic and the low-frequency viscoelastic characteristic calculated in Step S8 (Step S10). By calculating this correlation, the characteristic of the measurement sample S can be made clear. When it has been calculated in Step S10 that the correlation between the high-frequency viscoelastic characteristic and the low-frequency viscoelastic characteristic is low (e.g., equal to or lower than a predetermined threshold), for example, this means the change in the characteristics of the measurement sample S, which has not been made clear by the measurement of only the low-frequency viscoelastic characteristic, becomes clear by measuring the high-frequency viscoelastic characteristic of the measurement sample S. Accordingly, the new discovery can be made regarding the characteristic of the measurement sample S. When the feature amount extracted in Step S9 uses both the low-frequency viscoelastic characteristic and the high-frequency viscoelastic characteristic, the change in the feature amount can be known from the correlation between the low-frequency viscoelastic characteristic and the high-frequency viscoelastic characteristic, not the low-frequency viscoelastic characteristic alone or the high-frequency viscoelastic characteristic alone.

The operating unit 35 extracts influences of an external field (e.g., an electric field, a magnetic field, or an electromagnetic field) on the high-frequency viscoelastic characteristic (feature amount generated in accordance with the presence of the external field) based on the correlation calculated in Step S10 (Step S11). The data that has been calculated or extracted in Steps S8 to S11 may be displayed on or output to the display/output unit 38.

As stated above, the viscoelastic characteristic measurement apparatus 1 measures the high-frequency viscoelastic characteristic using the sound wave in a state in which the measurement sample S is deformed using the rheometer. It is therefore possible to measure the high-frequency viscoelastic characteristic in a state in which the actual use state of the measurement sample S is appropriately reflected. In a normal travelling state, for example, it is assumed that the tire is deformed at a frequency of several Hz to 100 Hz. Accordingly, when the high-frequency viscoelastic characteristic of the tire or the material of the tire is measured as the measurement sample S, the viscoelastic characteristic measurement apparatus 1 is able to measure the high-frequency viscoelastic characteristic when the tire travels more accurately by vibrating the tire or the material of the tire at a frequency of several Hz to 100 Hz.

Further, the viscoelastic characteristic measurement apparatus 1 is able to measure the low-frequency viscoelastic characteristic of the measurement sample S such as rotation or vibrations as well. Accordingly, the viscoelastic characteristic measurement apparatus 1 is able to calculate the correlation between the high-frequency viscoelastic characteristic and the low-frequency viscoelastic characteristic and the influences of the external field on the high-frequency viscoelastic characteristic.

[Second Embodiment]

Hereinafter, with reference to the drawings, a second embodiment of the present invention will be described. In the second embodiment, an example of calculating the high-frequency viscoelastic characteristic of the measurement sample S using a bottom face reflection method, not the surface reflection method, will be described. The bottom face reflection method is a method of measuring the high-frequency viscoelastic characteristic based on the reflected sound wave generated as a result of reflection of the incident sound wave in the surface of the measurement sample when the incident sound wave is made incident on the measurement sample S (hereinafter this wave will be referred to as a first reflected sound wave) and a reflected sound wave generated as a result of reflection of the incident sound wave that has transmitted the surface of the measurement sample in the surface opposite to the above surface (hereinafter this wave will be referred to as a second reflected sound wave) (see, for example, Patent Literature 1).

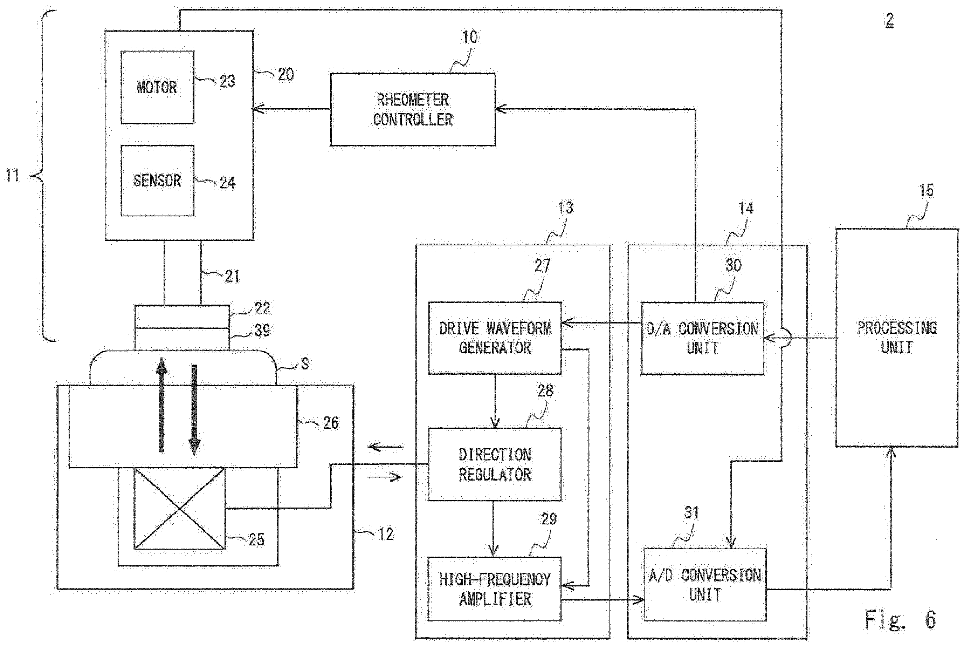

Fig. 6 is a block diagram showing a configuration example of a viscoelastic characteristic measurement apparatus 2 according to the second embodiment. The viscoelastic characteristic measurement apparatus 2 according to the second embodiment is different from the viscoelastic characteristic measurement apparatus 1 according to the first embodiment in that a reflection member 39 is additionally provided in the viscoelastic characteristic measurement apparatus 2 according to the second embodiment. Since the other components of the viscoelastic characteristic measurement apparatus 2 are similar to those in the first embodiment, the descriptions thereof will be omitted.

The reflection member 39 is provided in such a way that it contacts the surface of the measurement sample S opposite to the surface of the measurement sample S on which the incident sound wave is made incident (that is, surface that is opposed to the delay member 26 and the transducer 25). Specifically, the surface of the reflection member 39 that contacts the measurement sample S is parallel to the surface of the delay member 26 that contacts the measurement sample S. The reflection member 39 is provided between the measurement sample S and the annular plate 22. In this example, the reflection member 39 is fixed to the annular plate 22 and moves according to the movement of the axis 21. When the axis 21 rotates, for example, the reflection member 39 is also rotated. Accordingly, the reflection member 39 transmits the external force from the axis 21 to the measurement sample S. The reflection member 39 and the end part of the measurement sample S are open to the air.

The reflection member 39 is moved by hand of the measurer or automatically moved by a machine, which causes a change in a distance between the reflection member 39 and the delay member 26. The incident sound wave that has transmitted inside the measurement sample S is reflected in the reflection member 39. The second reflected sound wave reflected in the reflection member 39 is made incident on the delay member 26. The delay member 26 outputs the second reflected sound wave to the transducer 25 and the transducer 25 converts the second reflected sound wave into the electric signal and supplies the electric signal to the direction regulator 28. The direction regulator 28 supplies the electric signal to the high-frequency amplifier 29. The high-frequency amplifier 29 amplifies the high-frequency components of the electric signal and supplies the electric signal after the amplification to the processing unit 15.

Further, the acoustic impedance difference in the reflection member 39 is larger than that in the measurement sample S. Therefore, the incident sound wave emitted from the transducer 25 is efficiently reflected on the interface.

<Method of calculating High-Frequency Viscoelastic Characteristic (Bottom Face Reflection Method)>

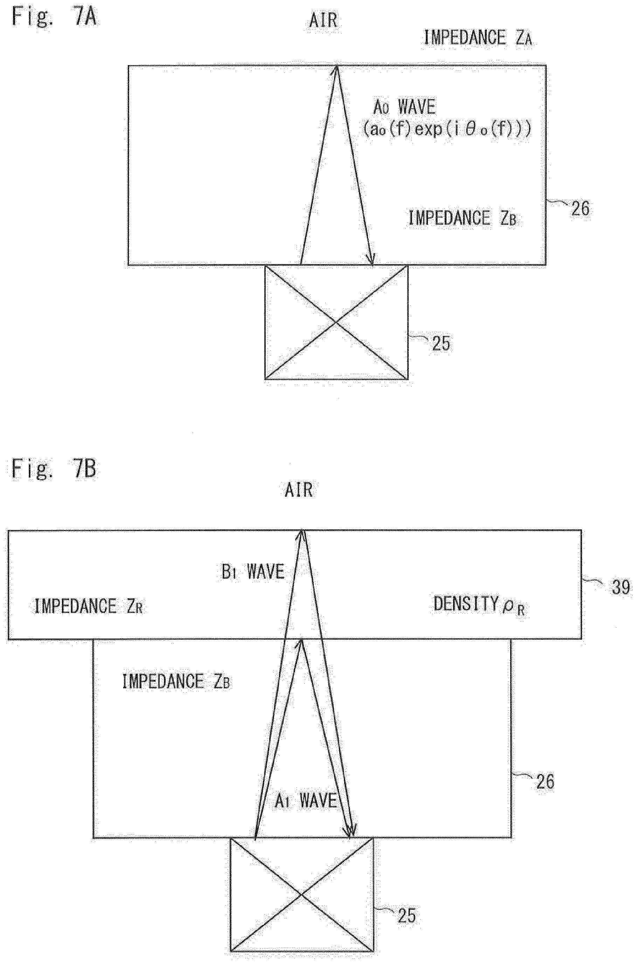

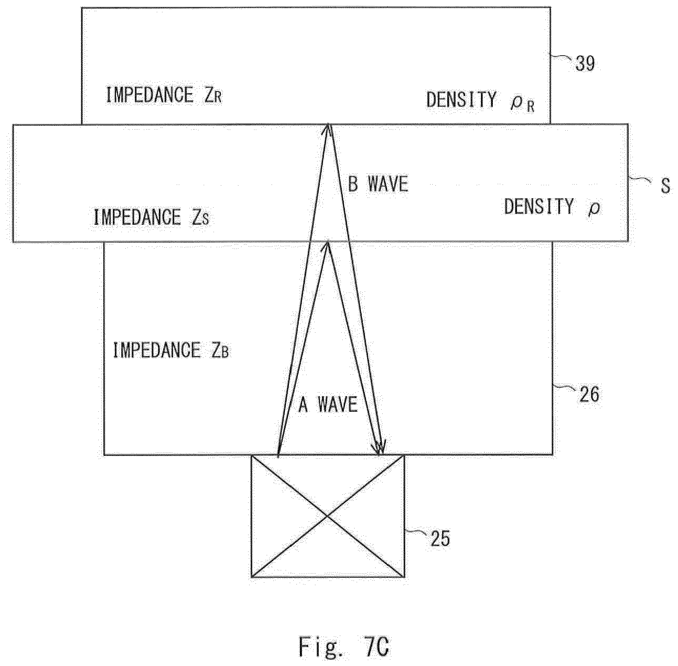

Figs. 7A, 7B, and 7C are diagrams describing a method of calculating the high-frequency viscoelastic characteristic using the bottom face reflection method. Figs. 7A and 7B are diagrams showing the reflection status of the incident sound wave when the reference value is acquired and Fig. 7C is a diagram showing the reflection status of the incident sound wave when the high-frequency viscoelastic characteristic of the measurement sample S is calculated. In the following description, an acoustic impedance indicating the propagation characteristic of the incident sound wave emitted from the transducer 25 of the sound wave emission unit 12 is used.

Referring first to Fig. 7A, the reference value will be described. In a state in which the delay member 26 is not in close contact with any one of the reflection member 39 and the measurement sample S, the transducer 25 emits the incident sound wave. Then the transducer 25 receives the reflected sound wave (hereinafter this wave is also referred to as an $A_0$ wave) that is generated in the interface between the delay member 26 and the air. In order to eliminate an error in an initial state, which occurs due to the presence of the delay member 26, the operating unit 35 acquires the incident sound wave emitted from the transducer 25 via the delay member 26 as the reference value.

As already described in the first embodiment, the acoustic impedance in the air is much smaller than the acoustic impedance of the delay member 26 and that of the measurement sample S. Therefore, the entire incident sound wave emitted from the transducer 25 is reflected on the interface between the delay member 26 and the air.

The $A_0$ wave received by the transducer 25 is expressed by $a_0(f)\exp(i\theta_0(f))$. The symbol i denotes an imaginary unit, $a_0(f)$ denotes an amplitude value (real value) at each frequency, and $\theta_0(f)$ denotes a phase (constant equal to 0 or larger) at each frequency. Then the operating unit 35 performs FFT processing on the time waveform of the reflected sound wave received by the transducer 25 to calculate the amplitude value $a_0(f)$ and the phase $\theta_0(f)$ at each frequency.

With reference next to Fig. 7B, descriptions will be given. Upon receiving a reflection member reference command from the input unit 34, the operating unit 35 acquires data of the thickness and the density of the reflection member 39. This data may be input from the input unit 34 at a timing the same as the timing when the emission instruction is input or may be stored in the storage unit 37 in advance. In the delay member 26 shown in Fig. 7B, the surface of the delay member 26 with which the reflection member 39 is in close contact is on the opposite side of the surface of the delay member 26 with which the transducer 25 is in close contact. When the incident sound wave is emitted from the transducer 25, the incident sound wave is reflected on the interface between the delay member 26 and the reflection member 39, which causes a reflected sound wave (hereinafter this wave will also be referred to as an $A_1$ wave). Further, since the incident sound wave is reflected also in the interface between the reflection member 39 and the air, another reflected sound wave (hereinafter this wave will also be referred to as a $B_1$ wave) is generated.

In this case, the operating unit 35 detects the time delay between the $A_1$ wave and the $B_1$ wave. The operating unit 35 measures a delay time $\Delta T_R$ between the $A_1$ wave and the $B_1$ wave based on the time waveforms of the $A_1$ wave and the $B_1$ wave received from the transducer 25.

When the thickness of the reflection member 39 is denoted by $h_R$ and the density of the reflection member 39 is denoted by $\rho_R$, the acoustic impedance $Z_R$ of the reflection member 39 is as follows.

$$Z_R = 2h_R\rho_R/\Delta T_R \cdots (13)$$

From the aforementioned discussion, the operating unit 35 acquires the acoustic impedance $Z_R$ of the reflection member 39 based on the data of the thickness $h_R$ of the reflection member 39 and the density $\rho_R$ of the reflection member 39 acquired in advance and the measured delay time $\Delta T_R$ using Expression (13). The operating unit 35 performs the aforementioned calculation to calculate the reference value to calculate the high-frequency viscoelastic characteristic.

With reference next to Fig. 7C, the calculation of the high-frequency viscoelastic characteristic of the measurement sample S will be described. When the lose tangent of the measurement sample S is calculated, the measurement sample S is arranged in such a way that one surface thereof is in close contact with the delay member 26 and another surface thereof is in close contact with the reflection member 39. Upon receiving the instruction to emit the sound wave from the input unit 34, the operating unit 35 outputs the electric signal to the sound wave signal generation unit 13. According to this processing, the incident sound wave is emitted to the measurement sample S. The first and second reflected sound waves that are generated as a result of the reflection of the incident sound wave in the measurement sample S are stored in the time data memory unit 36, similar to the first embodiment.

Upon receiving the instruction to emit the incident sound wave from the input unit 34, the operating unit 35 acquires the data of the thickness and the density of the measurement sample S. This data may be input from the input unit 34 at a timing the same as the timing when the emission instruction is received or may be stored in the storage unit 37 in advance.

When the transducer 25 emits the incident sound wave in accordance with the electric signal of the drive waveform generator 27, the incident sound wave is reflected on the interface between the delay member 26 and the measurement sample S, which causes generation of a reflected sound wave (hereinafter this wave will also be referred to as an A wave). Further, since the incident sound wave is reflected on the interface between the measurement sample S and the reflection member 39, a reflected sound wave (hereinafter this wave will also be referred to as a B wave) is generated. The operating unit 35 detects the time delay between the A wave and the B wave and also acquires the amplitude value and the phase of each of the A wave and the B wave at each frequency. More specifically, the operating unit 35 measures the delay time $\Delta T$ between the A wave and the B wave based on the time waveform from the transducer 25.

When the thickness of the measurement sample S is denoted by h and the density of the measurement sample S is denoted by ρ, the acoustic impedance Z of the measurement sample S is as follows.

$$Z_S = 2h\rho/\Delta T \cdots (14)$$

Using Expression (14), the reflectance R of the reflected sound wave in the interface between the measurement sample S and the reflection member 39 is expressed as follows.

$$R = (Z_R - Z_S)/(Z_R + Z_S) = (Z_R - 2h\rho/\Delta T)/(Z_R + 2h\rho/\Delta T) \cdots (15)$$

From the above description, the operating unit 35 acquires, based on the data of the thickness h of the measurement sample S and the density p of the measurement sample S, and the acoustic impedance $Z_R$ of the reflection member 39 that have been acquired in advance, and the delay time $\Delta T$ that has been measured, the reflectance R of the reflected sound wave using Expression (15).

Now, the A wave received by the transducer 25 is expressed by $a(f)\exp(i\theta_A(f))$ and the B wave received by the transducer 25 is expressed by $b(f)\exp(i\theta_B(f))$, where i denotes an imaginary unit, $a(f)$ and $b(f)$ are amplitude values (real values) of the A wave and the B wave at each frequency, and $\theta_A(f)$ and $\theta_B(f)$ are phases of the A wave and the B wave at each frequency. The symbols $\theta_A(f)$ and $\theta_B(f)$ are real numbers equal to or larger than 0.

The operating unit 35 reads out the time waveform data of the A wave and the B wave stored in the time data memory unit 36 and measures the delay time of the second reflected sound wave with respect to the first reflected sound wave. Further, the operating unit 35 performs FFT processing on the time waveform of the A wave received by the transducer 25 to acquire the amplitude value $a(f)$ and the phase $\theta_A(f)$ of the A wave at each frequency. In a similar way, the operating unit 35 performs FFT processing on the time waveform of the B wave received by the transducer 25 to acquire the amplitude value $b(f)$ and the phase $\theta_B(f)$ of the B wave at each frequency.

Using the amplitude values of the $A_0$ wave, the A wave, and the B wave at each frequency and the calculated reflectance R, an attenuation coefficient $\alpha(f)$ of the incident sound wave is expressed by the following expression.

$$\alpha(f) = (1/2h)\ln(R(a_0(f)^2 - a(f)^2)/a_0(f)b(f)) \cdots (16)$$

Further, using the amplitude values and the phases of the A wave and the B wave at each frequency, a phase velocity Vp(f) of the incident sound wave is expressed by the following expression.

$$V_P(f) = 2h \times 2\pi f/(\theta_B - \theta_A + 2\pi f\Delta T + 2N\pi) \cdots (17)$$

Here, N is an arbitrary positive number.

Using the attenuation coefficient $\alpha(f)$ and the phase velocity Vp(f) derived by the above expressions, the loss tangent $\tan\delta(f)$ can be expressed by the following expression.

$$\tan\delta(f) = \alpha(f) \times Vp(f)/\pi f \cdots (18)$$

Further, the storage elastic modulus E'(f) and the loss elastic modulus E"(f) can be expressed as the following expressions.

$$E'(f) = \rho Vp^2(f) \cdots (19)$$

$$E''(f) = 2\alpha\rho Vp^3(f)/\omega = \alpha Vp(f)E'(f)/\pi f \cdots (20)$$

The operating unit 35 performs calculations of Expressions (16) to (20) to calculate the high-frequency viscoelastic characteristic of the measurement sample S. The operating unit 35 further outputs the high-frequency viscoelastic characteristic of the measurement sample S that has been calculated to the display/output unit 38.

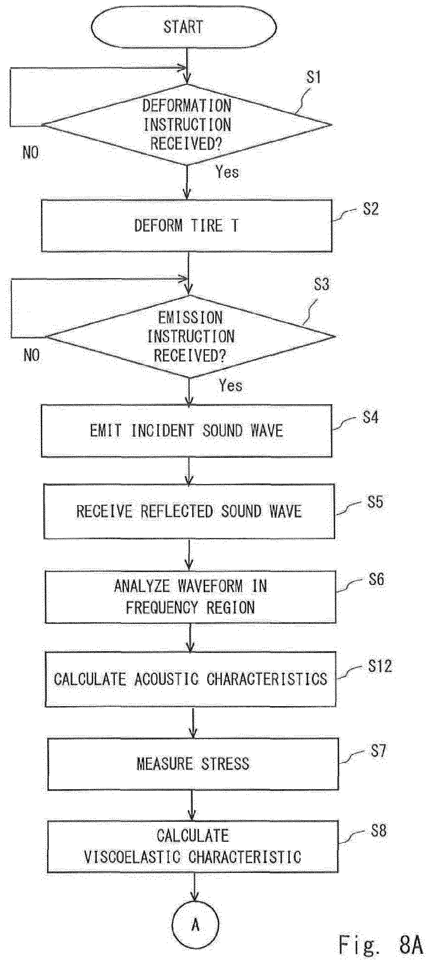

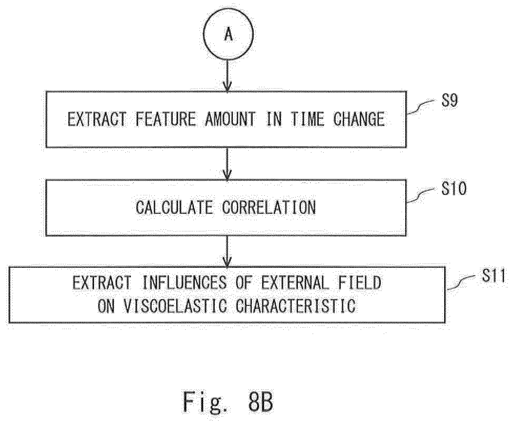

Figs. 8A and 8B are flowcharts showing one example of the processing of the viscoelastic characteristic measurement apparatus 2. In the following description, with reference to Figs. 8A and 8B, the whole processing of the viscoelastic characteristic measurement apparatus 2 will be described.

Since Steps S1 to S6 in Fig. 8A are the same as the processing of Steps S1 to S6 in Fig. 5A, the descriptions thereof will be omitted.

In Step S12 of Fig. 8A, the attenuation coefficient $\alpha(f)$ and the phase velocity $Vp(f)$, which are acoustic characteristics of the measurement sample S, are calculated. The method of calculating the acoustic characteristics has been stated above. In Step S8 of Fig. 8A, the operating unit 35 calculates the high-frequency viscoelastic characteristic of the measurement sample S based on the attenuation coefficient $\alpha(f)$ and the phase velocity $Vp(f)$. The calculation of the low-frequency viscoelastic characteristic of the measurement sample S is similar to Step S8 shown in Fig. 5A.

Since Steps S7 to S11 of Figs. 8A and 8B are similar to the processing of Steps S7 to S11 of Figs. 5A and 5B, the descriptions thereof will be omitted. As described above, even when the bottom face reflection method is used, the high-frequency viscoelastic characteristic can be measured using the sound wave in the state in which the rheometer deforms the measurement sample S.

[Third Embodiment]

Hereinafter, with reference to the drawings, a third embodiment of the present invention will be described. In the third embodiment, an example of calculating the high-frequency viscoelastic characteristic of the measurement sample S using a transmission method, not the surface reflection method or the bottom face reflection method, will be described. In the transmission method, a transducer that emits the incident sound wave and a transducer that receives the transmitted sound wave are separately provided (see, for example, Patent Literature 1).

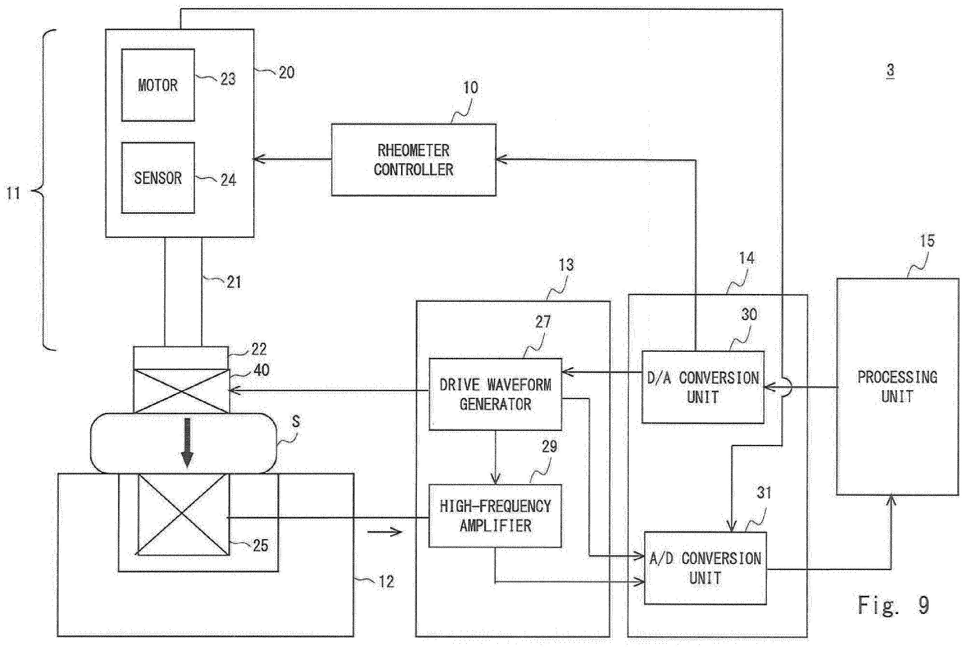

Fig. 9 is a block diagram showing a configuration example of a viscoelastic characteristic measurement apparatus 3 according to a third embodiment. The viscoelastic characteristic measurement apparatus 3 according to the third embodiment is different from the viscoelastic characteristic measurement apparatus 1 according to the first embodiment in that a transducer 40 is newly provided and the delay member 26 and the direction regulator 28 are not provided in the viscoelastic characteristic measurement apparatus 3 according to the third embodiment. Since the other components of the viscoelastic characteristic measurement apparatus 3 are similar to those in the first embodiment, the descriptions thereof will be omitted.

The transducer 40 is provided in such a way that it contacts the surface of the measurement sample S which is on the side opposite to the surface that contacts the transducer 25. More particularly, the surface of the transducer 40 that contacts the measurement sample S is parallel to the surface of the transducer 25 that contacts the measurement sample S. The transducer 40 is provided between the measurement sample S and the annular plate 22. The transducer 40 converts the electric signal of the drive waveform generated in the drive waveform generator 27 into the sound wave. The sound wave after the conversion is emitted to the measurement sample S. After the sound wave is made incident, the sound wave that has transmitted the measurement sample S (transmitted sound wave) is input to the transducer 25. The processing performed by the transducer 25 is the same as that in the first embodiment.

Further, the electric signal of the drive waveform generated by the drive waveform generator 27 is also output to the A/D conversion unit 31. The A/D conversion unit 31 performs A/D conversion of the electric signal. The electric signal that has been A/D converted is stored in the time data memory unit 36. According to this processing, the time waveform data of the incident sound wave is stored in the time data memory unit 36. Further, the time waveform data of the transmitted sound wave is also stored in the time data memory unit 36, similar to the first embodiment.

In the third embodiment, when the measurer inputs the instruction to emit the incident sound wave to the input unit 34, the incident sound wave is emitted to the measurement sample S. The details of this processing have been stated above. The time data memory unit 36 stores the time waveform data of the incident sound wave and the time waveform data of the transmitted sound wave. The operating unit 35 reads out the time waveform data of the incident sound wave and the time waveform data of the transmitted sound wave stored in the time data memory unit 36 and performs FFT processing on the two pieces of time waveform data that have been read out. According to this processing, the operating unit 35 acquires the amplitude value and the phase at each frequency. Next, the operating unit 35 compares the amplitude value and the phase of each of the incident sound wave and the transmitted sound wave at each frequency to calculate the high-frequency viscoelastic characteristic of the measurement sample S. The operating unit 35 then outputs the high-frequency viscoelastic characteristic of the measurement sample S that has been calculated to the display/output unit 38. Since the other processings executed by the respective elements of the viscoelastic characteristic measurement apparatus 3 are similar to those in the first embodiment, the descriptions thereof will be omitted. The viscoelastic characteristic measurement apparatus 3 may have a configuration in which the transducer 25 emits the incident sound wave and the transducer 40 receives the transmitted sound wave.

In the following description, measurement examples executed by the rheometer unit 11 using the methods described in the first to third embodiments will be described.

[Measurement Example 1]

The rheometer unit 11 is able to rotate the measurement sample S about a rotation axis at a predetermined frequency. This rotation axis is an axis (e.g., axis 21) parallel to the direction to which the incident sound wave is emitted. According to this configuration, the measurement sample S rotates at a predetermined frequency in a direction vertical to the direction to which the incident sound wave is emitted. In this way, torsional deformation may occur in the measurement sample S.

[Measurement Example 2]

The rheometer unit 11 applies a tensile force or a compressive force to the measurement sample S, whereby deformation (stretch or contract) occurs in the measurement sample S. Further, the rheometer unit 11 may cause deformation (stretch and contract) in one direction at a predetermined cycle by vibrating the measurement sample S at a predetermined cycle. The rheometer unit 11 may make the measurement sample S vibrate at a predetermined frequency in a direction parallel to the direction to which the incident sound wave is emitted. The direction to which the tensile force or the compressive force is added is parallel to the direction to which the incident sound wave is emitted.

[Measurement Example 3]

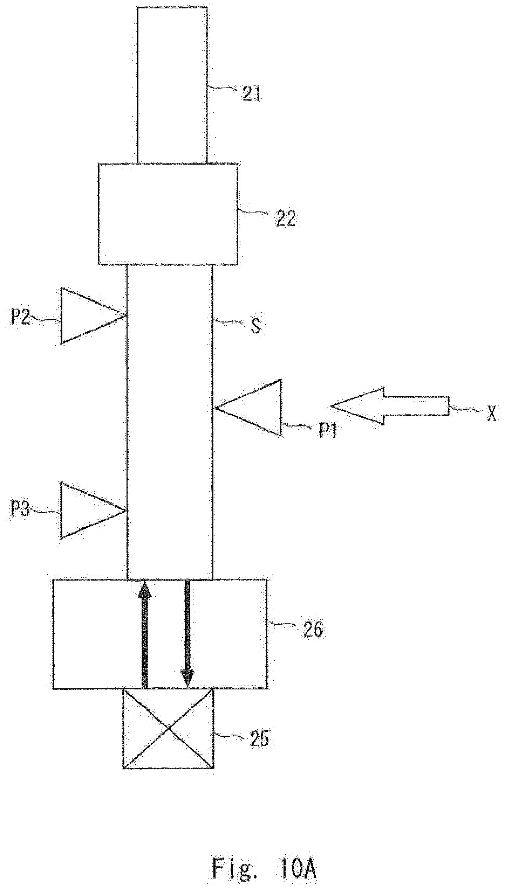

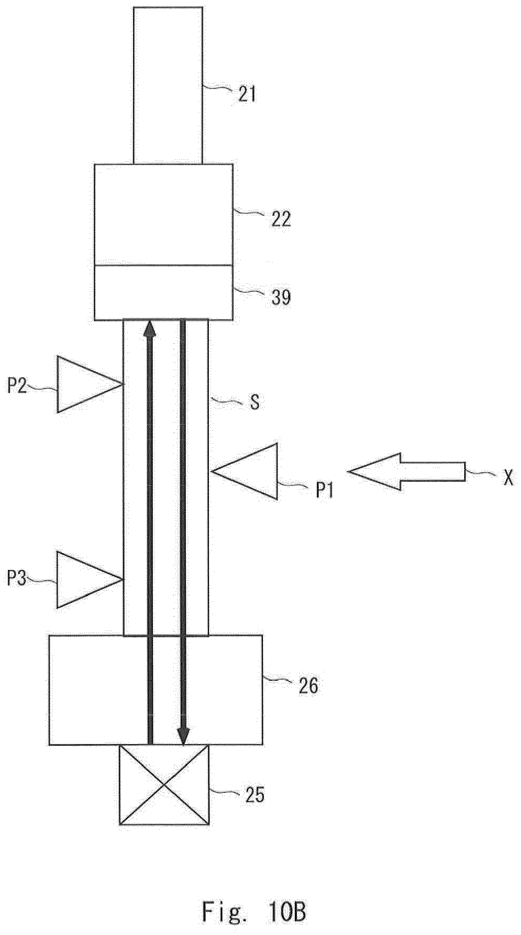

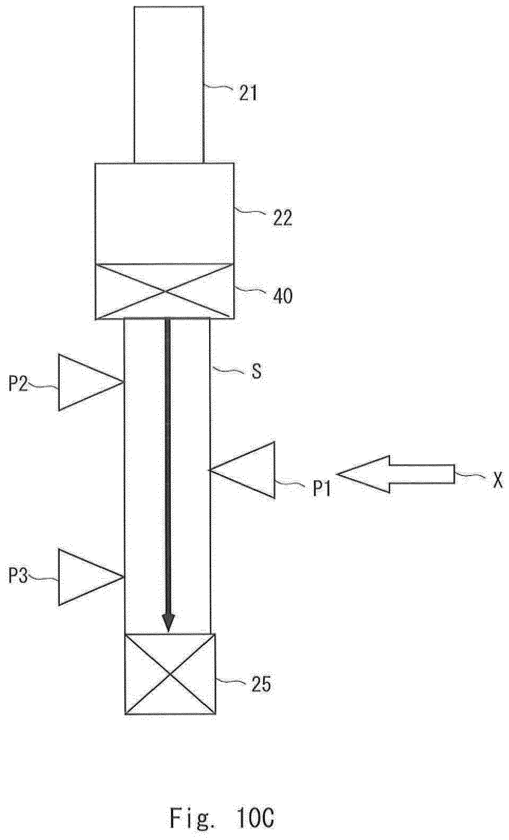

Figs. 10A to 10C are diagrams showing examples of measuring the high-frequency viscoelastic characteristic in a case in which a bending force is applied to the measurement sample S. Fig. 10A is a diagram showing a case in which the high-frequency viscoelastic characteristic is measured using the surface reflection method, Fig. 10B is a diagram showing a case in which the high-frequency viscoelastic characteristic is measured using the bottom face reflection method, and Fig. 10C is a diagram showing a case in which the high-frequency viscoelastic characteristic is measured using the transmission method. Figs. 10A to 10C show only the components around the transducer and the measurement sample S and do not show the other components. In Figs. 10A and 10B, the incident sound wave is made incident from bottom to top and in Fig. 10C, the incident sound wave is made incident from top to bottom.
In Figs. 10A to 10C, a load is applied to the measurement sample S at a predetermined cycle from a point P1 in the direction of an arrow X. That is, the force toward the lateral direction (left-side direction in Figs. 10A to 10C) is added to the measurement sample S. Further, the measurement sample S is supported at points P2 and P3. The points P2 and P3 contact the measurement sample S on the side opposite to the point P1 with respect to the measurement sample S (that is, on the left side in Figs. 10A to 10C). The point P2 contacts a part in the vicinity of an end part of the measurement sample S and the point P3 contacts a part in the vicinity of another end part of the measurement sample S opposite to the part that contacts the point P2. The points P2 and P3 prevent the whole measurement sample S from being shifted in the lateral direction. According to this configuration, only the part around the point P1 of the measurement sample S is bent in the direction in which the stress is applied at a predetermined cycle. It is therefore possible to cause a bending deformation in the measurement sample. By applying the force in the direction of the arrow X (applying sine wave vibrations) to the measurement sample S from the point P1 at a predetermined cycle, only the part around the point P1 can be bent in the direction in which the stress is applied at a predetermined cycle.

[Measurement Example 4]

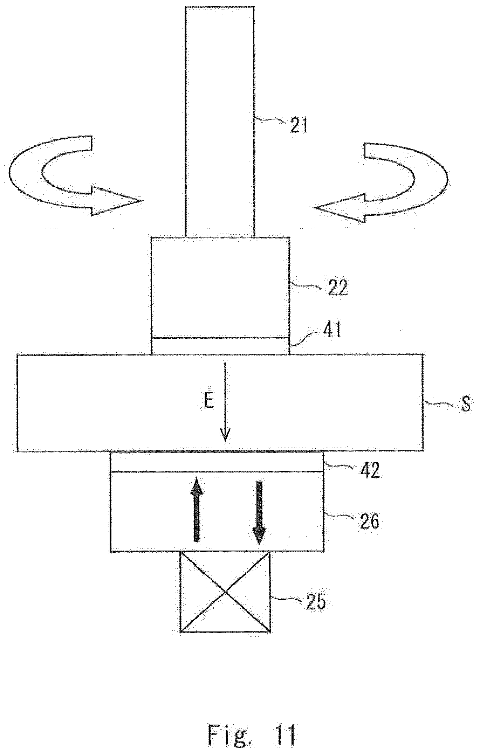

Fig. 11 is a diagram showing a measurement example of the high-frequency viscoelastic characteristic when the measurement sample S is rotated about the rotation axis at a predetermined frequency and an electric field is applied to the measurement sample S. In Fig. 11 the high-frequency viscoelastic characteristic is measured using the surface reflection method and the incident sound wave is made incident from top to bottom in Fig. 11. Fig. 11 shows only the components around the transducer and the measurement sample S and does not show the other components. One example of the measurement sample S in Measurement Example 4 is Electro-Rheological (ER) fluid, liquid crystal, or a piezoelectric material.
In Fig. 11, an electrode 41 is provided between the annular plate 22 and the measurement sample S and an electrode 42 is provided between the delay member 26 and the measurement sample S. When an electric field E is applied from the electrode 41 to the electrode 42, the electric field is applied to the measurement sample S in a direction parallel to the direction to which the emitted sound wave is emitted. The rheometer controller 10 is able to adjust the strength of the electric field to be applied to the electrodes 41 and 42. The electrodes 41 and 42 and the rheometer controller 10 form an electric field applying unit. When the strength of the electric field applied by the measurer is specified from the input unit 34 of the processing unit 15, the operating unit 35 acquires the information. The operating unit 35 controls the rheometer controller 10 to cause the rheometer controller 10 to adjust the strength of the electric field as specified based on the information on the strength of the electric field that has been acquired. According to the above processing, it is possible to measure the high-frequency viscoelastic characteristic by changing the strength of the electric field to be applied to the measurement sample S. When the measurement sample S is ER fluid, the viscosity is changed in accordance with the electric field from the external field, whereby it is possible to acquire data of different high-frequency viscoelastic characteristics by changing the strength of the electric field.

In Measurement Example 1, random rotation or vibrations may be added to the measurement sample S in place of the rotation or the vibrations at a predetermined frequency. As described above, however, in order to calculate the low-frequency viscoelastic characteristic, it is preferable to add since wave rotation or sine wave vibrations at a predetermined frequency to the measurement sample S.

In Measurement Example 4, by arranging magnets in place of electrodes, it is possible to measure the high-frequency viscoelastic characteristic by applying the magnetic field to the measurement sample S. In this case, one example of the measurement sample S is Magneto-Rheological (MR) fluid. Further, by arranging a radio wave emission source that emits radio waves in place of the electrodes, the high-frequency viscoelastic characteristic can be measured in a state in which the radio waves are emitted to the measurement sample S. In Measurement Example 4, the measurement sample S may not be rotated or a deformation that makes the measurement sample S vibrate, stretched, or contracted may be applied to the measurement sample S.

In Measurement Example 1, the measurement sample S rotates about the rotation axis parallel to the direction to which the incident sound wave is emitted. However, the rotation axis may not be parallel to the direction to which the incident sound wave is emitted. That is, the emission direction of the incident sound wave and the rotation axis may form a predetermined angle. In a similar way, in Measurement Example 2, the direction in which the tensile force or the compressive force is applied may not be parallel to the direction to which the incident sound wave is emitted. In Measurement Example 3 as well, the direction in which the bending force is applied may not be vertical to the direction in which the incident sound wave is emitted to the measurement sample S. In Measurement Example 4 as well, the direction in which the electric field is applied may not be parallel to the direction to which the emitted sound wave is emitted.

As described above, the parameters of the rotation and the vibration in Measurement Examples 1 to 4 can be set by the measurer. Further, the modified forms of the measurement sample S shown in the first to fourth modified examples can be combined as appropriate.

[Fourth Embodiment]

In the second embodiment, the high-frequency viscoelastic characteristic of the measurement sample S is calculated using the bottom face reflection method. Since the reflection member 39 and the measurement sample S contact each other in the air, the measurement sample S may be scattered or air bubbles may be mixed into the measurement sample S due to a centrifugal force caused by the rotation of the rheometer unit 11. When the measurement sample S is liquid, in particular, a change in the shape of the measurement sample S may occur. Such a change in the shape of the measurement sample S may cause an error in the measurement value of the measurement sample S, whereby the low-frequency and high-frequency viscoelastic characteristics may not be accurately measured.

In the fourth embodiment, in order to solve the above problem, the reflection member 39 and the measurement sample S are enclosed so that, even when a centrifugal force is applied to the measurement sample S, the change in the shape of the measurement sample S can be suppressed.

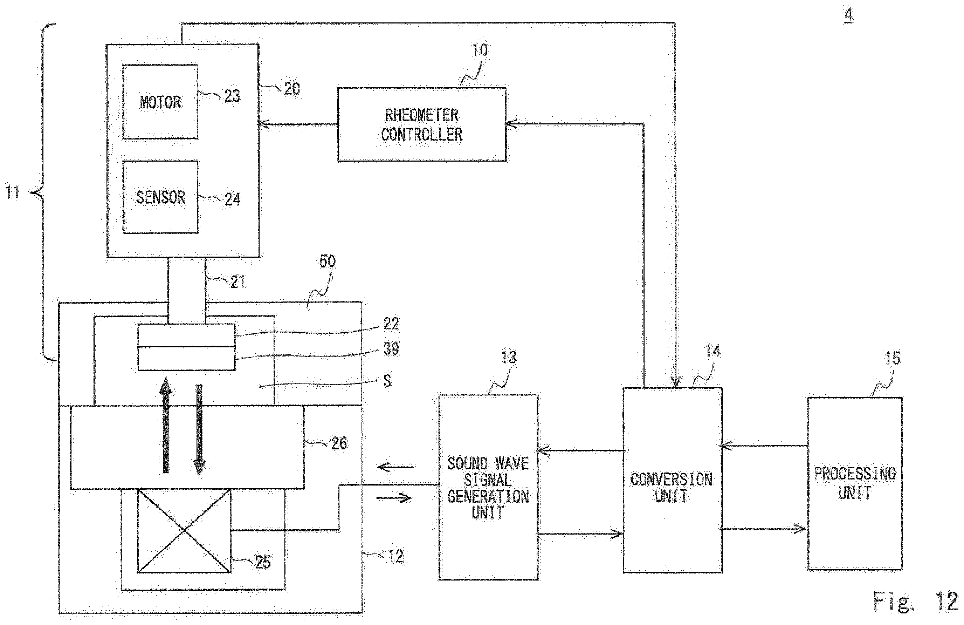

Fig. 12 is a block diagram showing a configuration example of a viscoelastic characteristic measurement apparatus 4 in a case in which the measurement sample S is liquid. In Fig. 12, a casing structure 50 encloses the annular plate 22, the reflection member 39, and the measurement sample S in a state in which there is little air inside therein, which suppresses air inclusion into the space between the reflection member 39 and the measurement sample S. The reflection member 39 is opposed to the delay member 26 with the measurement sample S interposed therebetween. Since the other components of the viscoelastic characteristic measurement apparatus 4 are similar to those of the viscoelastic characteristic measurement apparatus 2, the descriptions thereof will be omitted. Accordingly, even when the rheometer unit 11 rotates and the centrifugal force occurs, the measurement sample S is kept enclosed in the casing structure 50, whereby the change in the shape of the measurement sample S can be suppressed.

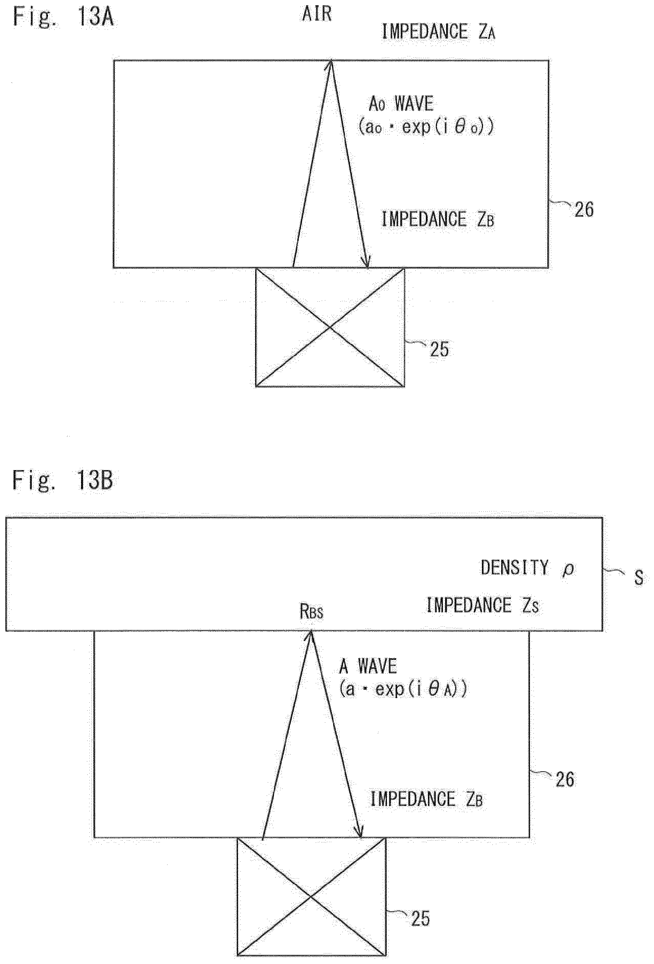

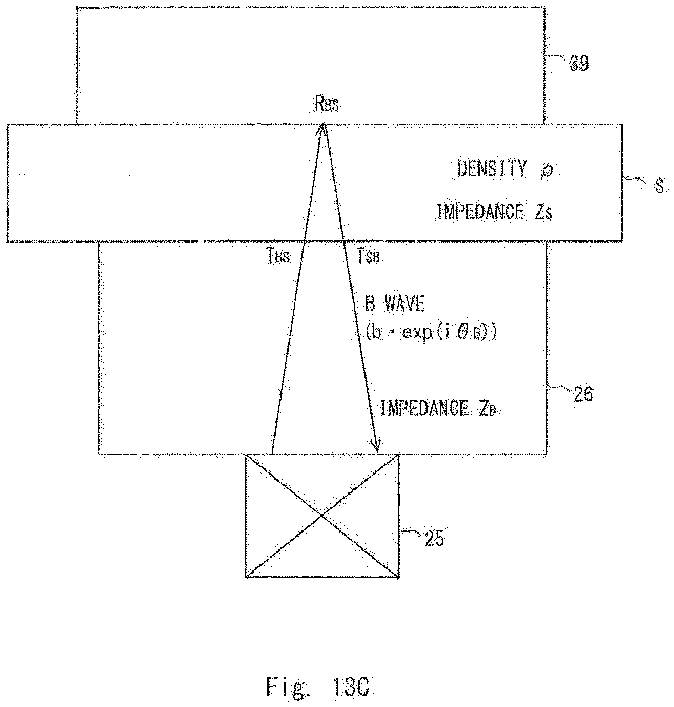

Further, in the fourth embodiment, another method of calculating the attenuation coefficient $\alpha$ of the incident sound wave will be further proposed. Figs. 13A to 13C are diagrams for describing a method of calculating the high-frequency viscoelastic characteristic using the bottom face reflection method. Fig. 13A is a diagram showing a situation in which the sound wave is made incident on the delay member 26 in a state in which the measurement sample S and the reflection member 39 are not provided. Fig. 13B is a diagram showing a situation in which the sound wave is made incident on the measurement sample S via the delay member 26 in a state in which the reflection member 39 is not provided. Further, Fig. 13C is a diagram showing a situation in which the sound wave is made incident on the measurement sample S via the delay member 26 in a state in which the reflection member 39 is provided.

In Figs. 13A to 13C, the acoustic impedance of the delay member 26 is denoted by $Z_B$ and the acoustic impedance of the measurement sample S is denoted by $Z_S$. Further, in the following calculations, influences of an attenuation, a delay, or multiple reflection of the sound wave in the interface between the delay member 26 and the measurement sample S are ignored.

The complex reflectance $R_{BS}$ of the incident wave from the delay member 26 to the measurement sample S can be expressed as shown in the following expression using $Z_B$ and $Z_S$.

$$R_{BS}=(Z_S-Z_B)/(Z_S+Z_B)\cdots(21)$$

From Expression (21), the acoustic impedance $Z_S$ can be expressed as follows.

$$Z_S=Z_B\cdot(1+R_{BS})/(1-R_{BS})\cdots(22)$$

The reflected sound wave ($A_0$ wave) generated in the interface between the delay member 26 and the air is compared with the reflected sound wave (A wave) generated in the interface between the delay member 26 and the measurement sample S. When the $A_0$ wave is expressed by the following expression

$$A_0=a_0\cdot\exp(i\theta_0)\cdots(23)$$

and the A wave is expressed by the following expression

$$A=a\cdot\exp(i\theta_A)\cdots(24),$$

the complex reflectance $R_{BS}$ can be expressed by the following expression.

$$R_{BS}=-(A/A_0)=(a\cdot\exp(i\theta_A))/(a_0\cdot\exp(i\theta_0))\cdots(25)$$

Now, the complex reflectance $R_{BS}$ will be defined as follows by separating the amplitude component from the phase component.

$$R_{BS}=-|R_{BS}|\exp(-i\theta_{BS})\cdots(26)$$

The amplitude component $|R_{BS}|$ and the phase component $\theta_{BS}$ are the following values.

$$|R_{BS}|=a/a_0\cdots(27)$$

$$\theta_{BS}(f)=\theta_0-\theta_A\cdots(28)$$

Next, a situation in which the sound wave is made incident on the measurement sample S in a state in which the reflection member 39 is provided will be discussed. In this case, the reflected sound wave (B wave) generated in the interface between the measurement sample S and the reflection member 39 is expressed by the following expression.

$$B=b\cdot\exp(i\theta_B)\cdots(29)$$

Further, the complex reflectance of the incident wave from the measurement sample S to the reflection member 39 determined based on the difference between the acoustic impedance $Z_S$ of the measurement sample S and the acoustic impedance $Z_R$ of the reflection member 39 is defined to be $R_{SR}$. Now, the B wave is expressed by the following expression.

$$b=a_0\cdot R_{SR}\cdot T_{BS}\cdot T_{SB}\cdot\exp(-2\alpha h)\cdots(30)$$

Now, h is a thickness of the measurement sample S and the item of exp(-2αh) in Expression (30) is an item indicating the attenuation by the measurement sample S. Further, $T_{BS}$ in Expression (30) is a transmittance from the delay member 26 to the measurement sample S, $T_{SB}$ is a transmittance from the measurement sample S to the delay member 26, and $T_{BS}$ and $T_{SB}$ will be defined as follows.

$$T_{BS} \equiv 1 - R_{BS} \cdots (31)$$

$$T_{SB} \equiv 1 - R_{SB} = 1 + R_{BS} \cdots (32)$$

$$R_{SB} \equiv -R_{BS} \cdots (33)$$

In this case, from Expressions (27), (31), and (32), the following expression is obtained.

$$T_{BS} \cdot T_{SB} = (1 - R_{BS}) \cdot (1 + R_{BS}) = 1 - R_{BS}^2 = 1 - (a/a_0)^2 = (a_0^2 - a^2)/a_0^2 \cdots (34)$$

Accordingly, by substituting Expression (34) into Expression (30), the attenuation coefficient $\alpha(f)$ of the incident sound wave is calculated as follows.

$$\alpha = (1/2h) \cdot \ln(R_{SR} \cdot ((a_0^2 - a^2)/a_0 \cdot b) \cdots (33)$$

This result is the same as the result of Expression (16).

When the acoustic impedance $Z_B$ of the delay member 26 and the acoustic impedance $Z_R$ of the reflection member 39 are the same value or substantially the same value (the value that can be regarded to be the same at the time of calculations), the following expression is obtained using Expressions (21) and (31).

$$R_{SR} = R_{SB} = -(Z_S - Z_B)/(Z_S + Z_B) = -R_{BS} \cdots (34)$$

Using Expression (27), the following expression is obtained.

$$R_{SR} = -R_{BS} = |R_{BS}| \exp(-i\theta_{BS}) \cdots (35)$$

When only the real part is considered since the phase component in (35) can be neglected, the real part is expressed by the following expression.

$$R_{SR} = -a/a_0 \cdots (36)$$

Therefore, the attenuation coefficient $\alpha(f)$ can be expressed by the following expression.

$$\alpha(f) = (1/2h) \cdot \ln((a^2 - a_0^2)a/a_0^2 \cdot b) \cdots (37)$$

As stated above, when the acoustic impedance $Z_B$ of the delay member 26 and the acoustic impedance $Z_R$ of the reflection member 39 are the same value, the attenuation coefficient $\alpha$ can be calculated without obtaining the complex reflectance $R_{SR}$, whereby it is possible to calculate the attenuation coefficient $\alpha$ more easily.

The aforementioned calculation can be executed even when the reflection member 39 and the measurement sample S are not enclosed as described in the second embodiment.

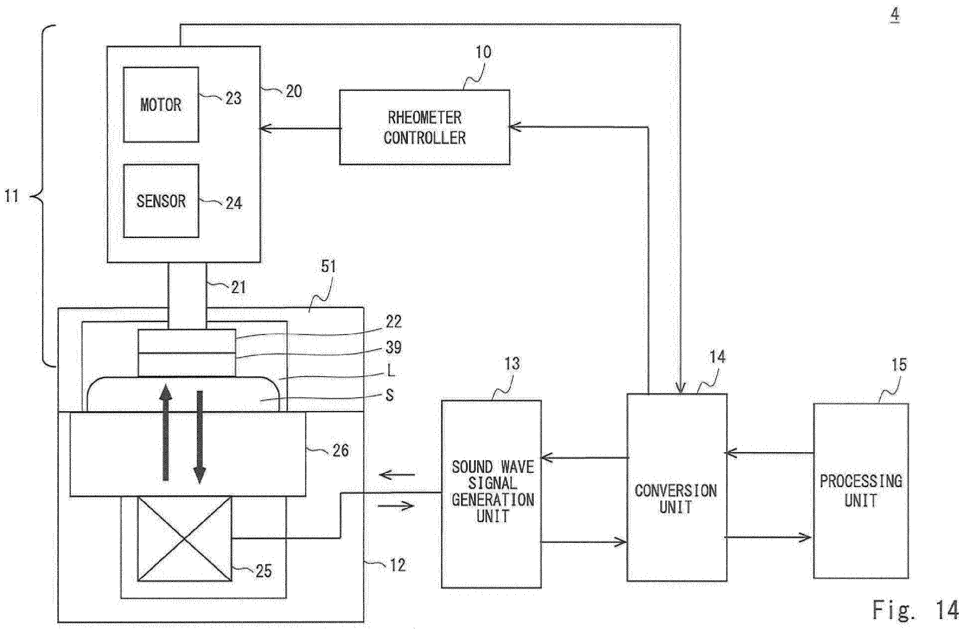

Further, even when the measurement sample S is solid, similar to the case shown in Fig. 12, the reflection member 39 and the measurement sample S can be enclosed. Fig. 14 is a block diagram showing a configuration example of the viscoelastic characteristic measurement apparatus 4 in a case in which the measurement sample S is solid. In Fig. 14, a casing structure 51 encloses the annular plate 22, the reflection member 39, and the measurement sample S. Further, the surface of the measurement sample S opposed to the reflection member 39 is a surface opposite to the surface of the measurement sample S opposed to the delay member 26. The casing structure 51 is filled with liquid L (e.g., water), which prevents air from entering the space between the reflection member 39 and the measurement sample S.

As shown in Figs. 12 and 14, in order to definitely prevent air from entering the space between the reflection member 39 and the measurement sample S, the vertical axis of the rheometer unit 11 and the sound wave emission unit 12 is preferably made to be sideways so that the axis corresponding to the direction in which the rheometer unit 11 and the sound wave emission unit 12 are arranged forms an angle (preferably an angle perpendicular) with respect to the gravity direction. In other words, the sound wave that is input from the transducer 25, propagated to the measurement sample S, and then reflected in the reflection member 39 preferably forms an angle (preferably an angle perpendicular) with respect to the gravity direction, not parallel to the gravity direction. Accordingly, even when air is mixed into the liquid measurement sample S shown in Fig. 12 or into the liquid L shown in Fig. 14, the mixed air does not accumulate around the surface in which the reflection member 39 and the measurement sample S contact each other, whereby it is possible to definitely prevent air from entering the space between the reflection member 39 and the measurement sample S.

As described above, in the viscoelastic characteristic measurement apparatus according to the first to fourth embodiments, it is possible to measure the behavior of the high-frequency viscoelastic characteristic when the measurement sample S is greatly deformed. As described above, when the high-frequency viscoelastic characteristic of a material such as rubber of a tire whose shape is deformed in use is measured, the high-frequency viscoelastic characteristic can be measured in a state in which the actual use state is appropriately reflected. When the measurement sample S is rotated or vibrated at a low frequency, in particular, the behavior of the low-frequency viscoelastic characteristic and that of the high-frequency viscoelastic characteristic can be measured at the same time in situ. It is therefore possible to compare the viscoelastic characteristics in the low frequency and the high frequency and the distortion dependencies thereof.

While the low frequency is a frequency of about, for example, 10 Hz and the high frequency is a frequency of about, for example, 1 MHz to 100 MHz, the ranges of the frequencies are not limited thereto. When a biological reaction or the like is measured by the viscoelastic characteristic measurement apparatus, for example, it may be efficient to measure the viscoelastic characteristic of the frequency lower than 1 MHz as a high frequency in a state in which stress is applied to the sample. In this case, a preferred high frequency is about 10 kHz to 100 MHz from the "effectiveness of wavelengths". The "effectiveness of wavelengths" means that there are limitations in the frequency and the wavelength of the sound wave since, when the wavelength of the sound wave used for a measurement becomes longer, the propagation length of the sample needs to be increased in order to separate waveforms to be measured. Further, in the study of ground, geology, earthquakes or the like, the viscoelastic characteristic is measured by detecting the propagation of the high frequency in a state in which a low-frequency stress is applied to the sample. In this case, a preferred high frequency is about 10 kHz to 100 kHz from the "effectiveness of wavelengths". Further, when a chemical reaction process is measured, the viscoelastic characteristic is measured by detecting the propagation of the high frequency during the process in a state in which a low-frequency stress is applied to the sample. In this case, a preferred high frequency is about 10 kHz to 100 MHz from the "effectiveness of wavelengths".

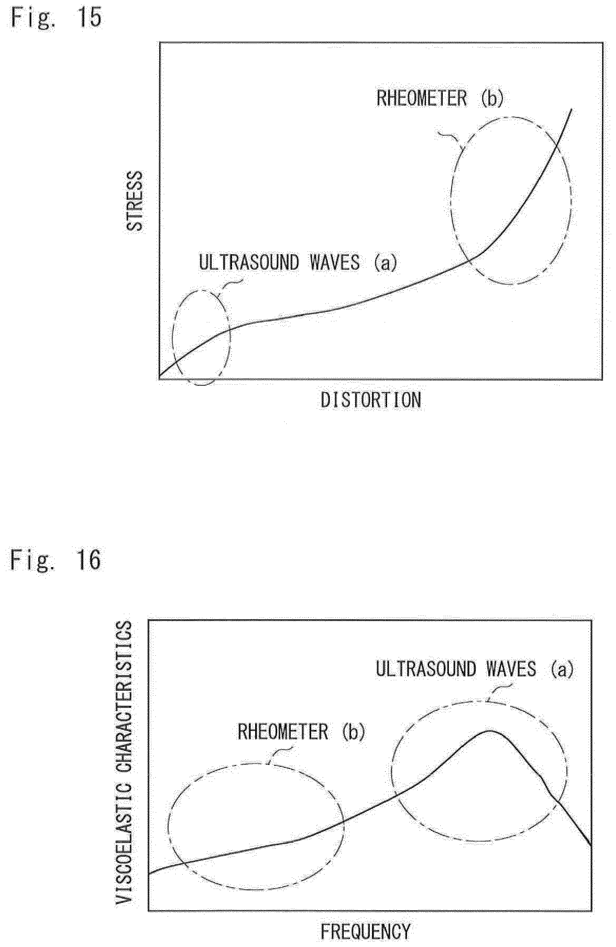

Fig. 15 is a graph showing one example of a relation between the distortion and the stress in the measurement sample having viscoelastic characteristics. In Fig. 15, the horizontal axis of the graph indicates the distortion and the vertical axis of the graph indicates the stress. Further, in Fig. 15, (a) shows a measurement range covered by the measurement that uses ultrasound waves and (b) shows a measurement range covered by the measurement that uses a rheometer. The measurement range in (a) is a state in which the distortion and the stress are small and the measurement range in (b) is a state in which the distortion and the stress are large. In the viscoelastic characteristic measurement apparatus according to the present invention, both ranges (a) and (b) can be measured.

Fig. 16 is a graph showing one example of a relation between the frequency and the viscoelastic characteristics in the measurement sample having the viscoelastic characteristics. In Fig. 16, the horizontal axis of the graph indicates the frequency and the vertical axis of the graph indicates the viscoelastic characteristics. Further, in Fig. 16, (a) shows a measurement range covered by the measurement that uses sound waves and (b) shows a measurement range covered by the measurement that uses a rheometer. The measurement range in (a) indicates the viscoelastic characteristics when the frequency and the viscoelastic characteristics are large and (b) indicates the viscoelastic characteristics when the frequency and the viscoelastic characteristics are small. In the viscoelastic characteristic measurement apparatus according to the present invention, both ranges (a) and (b) can be measured.

When the measurement sample S is a material such as dough or rubber and a reaction such as polymerization is performed in a chemical process in a state in which the measurement sample S is mixed, by applying rotation or vibrations to the measurement sample S, the aforementioned viscoelastic characteristic measurement apparatus is able to measure the change in the viscoelastic characteristics with time. Further, the reaction often proceeds from a surface of the measurement sample S. Accordingly, it is assumed that the surface physical properties of the measurement sample S (viscoelastic characteristics on the surface) differ from the physical properties of the bulk of the measurement sample S (the whole viscoelastic characteristics). The surface physical properties of the measurement sample S are calculated, for example, as a result of a measurement using the surface reflection method (in particular, measurement that uses ultrasound waves). Further, the physical properties of the bulk of the measurement sample S are calculated as a result of measuring the stress acting on the measurement sample S and the distortion of the measurement sample S when the measurement sample S is deformed using the rheometer. Accordingly, it is possible to appropriately determine the reaction progress by comparing data of these viscoelastic characteristics. Further, when the high-frequency viscoelastic characteristic is measured, the measurement sample S may be checked by Computed Tomography (CT) using a synthetic aperture method, whereby the physical properties of the measurement sample S can be stereoscopically understood. The synthetic aperture method means a technique of virtually synthesizing a cross sectional image of the measurement sample S using a phased array system when the measurement is performed by ultrasound waves. By means of such a technique, it is possible to determine the optimal degree of kneading of dough or rubber.

Further, in the first to fourth embodiments, the viscoelastic characteristic measurement apparatus includes a function in which the rheometer and the high-frequency viscoelasticity measurement apparatus are combined with each other. It is therefore possible to measure the low-frequency and high-frequency viscoelastic characteristics under the same or different distortions. It is therefore possible to measure, when the measurement sample S is rubber (material of the tire), the magnitude of a rolling resistance of the rubber by measuring, for example, the loss tangent $\tan\delta$ of the rubber in the low frequency when the load applied to the rubber is small (distortion applied to the rubber by the rheometer unit 11 is small). Further, by measuring the loss tangent $\tan\delta$ of the rubber in the low frequency when the load applied to the rubber is large (distortion applied to the rubber by the rheometer unit 11 is large), it is possible to measure the magnitude of a dry grip of the rubber. Further, by measuring the loss tangent $\tan\delta$ of the rubber in the high frequency when the load applied to the rubber is large (distortion applied to the rubber by the rheometer unit 11 is large), it is possible to measure the magnitude of a wet grip of the rubber. More specifically, by sending and receiving ultrasound waves from the transducer 25 provided in a chuck apparatus to rubber while applying torsional strain to the rubber by the rheometer unit 11, the loss tangent $\tan\delta$ in the high frequency can be measured.

The rolling resistance and the grip (dry grip and wet grip) of rubber stated above are parameters that change fuel economy of the tire. By measuring these parameters, it may be possible to efficiently or holistically evaluate a load-responsive smart material in which the material itself is changed so that it has the optimal property by torque generated in the load.

In general, when the loss tangent $\tan\delta$ of the rubber in the low frequency in a state in which a large load is applied is decreased for the purpose of reducing fuel economy of the tire, the loss tangent $\tan\delta$ of the rubber in the high frequency in a state in which a large load is applied is also decreased. Accordingly, in so-called fuel efficient tires, the performance of the wet grip is degraded.

From an analogy of the phenomenon in which the viscoelastic characteristic has distortion dependencies as seen in Payne effect, in rubber to which a large load (large distortion) is applied, the loss tangent $\tan\delta$ can be made larger than the original value (that is, the performance of the tire may be improved). Specifically, by employing a configuration in which the orientation of the aggregates, which are agglomerates of filler particles contained in rubber, does not irreversibly change due to a load distortion and the orientation of the aggregates is temporarily disconnected or moved, the viscoelastic characteristic of rubber can be changed according to the value of the load (distortion). The molecular weight of a side chain of the rubber and the molecular mass of the terminal are important elements that relate to the frequency dependency of the loss tangent $\tan\delta$.

As described above, the viscoelastic characteristic measurement apparatus according to the first to fourth embodiments can also be applied not only to the measurement of the loss tangent $\tan\delta$ in the low frequency and the high frequency but also to a study for optimizing the rolling resistance and the grip performance of rubber at the actual use temperature according to its application.

[Fifth Embodiment]

In the first to fourth embodiments, the operating unit 35 may calculate a frictional coefficient of the measurement sample S using the high-frequency viscoelastic characteristic of the measurement sample S that has been calculated. A frictional coefficient $\mu(f)$ of the measurement sample S, which is a function of the frequency f, can be expressed as the following expression using the aforementioned loss tangent $\tan\delta(f)$ and the storage elastic modulus E'(f).

$$\mu(f)=\alpha\times E'(f)^{n}\times\tan\delta(f)+\beta\cdots(37)$$

The symbols $\alpha(>0)$ and $\beta$ are unique constants that vary depending on the type of the tire (e.g., material of the tire) and n is a predetermined real number (e.g., n=-1/3). The expression to obtain the frictional coefficient $\mu(f)$ is a polynomial expression or a high-degree expression using $\tan\delta(f)$ other than Expression (37). The constants $\alpha$ and $\beta$ are values acquired by experiments or the like in advance and are stored in the storage unit 37.

[Sixth Embodiment]

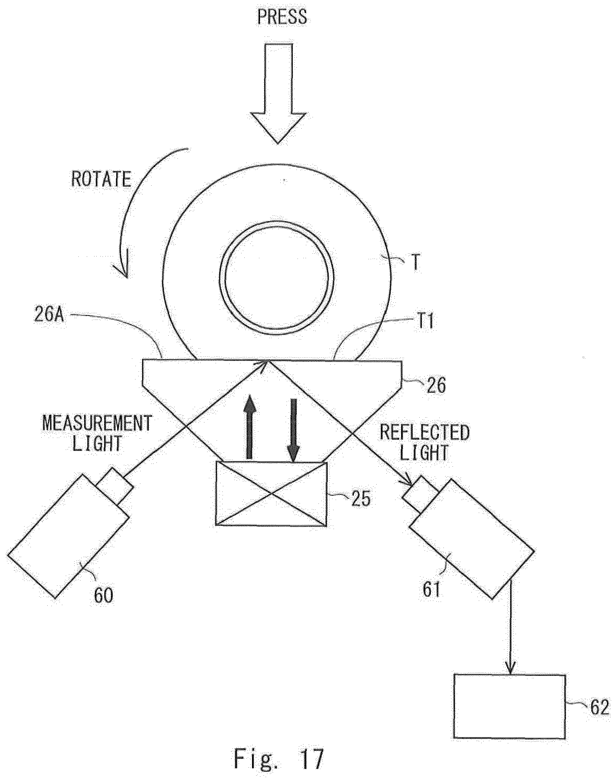

In a sixth embodiment, a configuration for optically measuring the measurement sample S, besides measuring the low-frequency and high-frequency viscoelastic characteristics, will be described. Fig. 17 is a diagram showing a partial configuration of a viscoelastic characteristic measurement apparatus 5 according to the sixth embodiment. Fig. 17 shows the tire T, which is the measurement sample S, the transducer 25, and the delay member 26. The viscoelastic characteristic measurement apparatus 5 further includes a light source 60, a high-speed CCD camera 61, and a processing unit 62 in addition to the elements of the viscoelastic characteristic measurement apparatus 1. The viscoelastic characteristic measurement apparatus 5 is an apparatus that calculates the high-frequency viscoelastic characteristic of the tire T by the surface reflection method. Since the other components of the viscoelastic characteristic measurement apparatus 5 are the same as those in the viscoelastic characteristic measurement apparatus 1, the descriptions thereof will be omitted.

Since a pressing force is applied to the tire T, a surface T1 of the tire T contacts a part of a surface 26A of the delay member 26. Then the tire T rotates while keeping in contact with the part of the surface 26A by the drive of the motor 23. Therefore, the shearing force generated due to the rotation and the frictional force generated due to the pressing force are generated in the surface T1. The tire T is fixed to keep in contact with the delay member 26 even when it is kept rotated (that is, it does not to move in the front-back direction). Further, the surface 26A is a surface of the delay member 26 opposite to the surface that the transducer 25 contacts.

The light source 60 irradiates the measurement light on the surface 26A via the delay member 26 and the high-speed CCD camera 61 detects a reflected light generated as a result of a reflection of the measurement light in the surface 26A via the delay member 26. The delay member 26 is transparent so that the measurement light from the light source 60 can be reflected in the surface 26A and the reflected light has an intensity that can be detected by the high-speed CCD camera 61.

When the measurement light is reflected in the surface 26A, it is possible to differentiate the area of the surface 26A that contacts the tire T from the area of the surface 26A that contacts another substance (in this example, air or water) by adjusting the incident angle of the measurement light that is made incident on the surface 26A from the light source 60. Since the reflectance of the delay member 26 and the tire T (rubber) is different from the reflectance of the delay member 26 and air or water, a critical angle $\theta_1$ in the interface between the delay member 26 and the tire T is different from a critical angle $\theta_2$ in the interface between the delay member 26 and air or water. Accordingly, in the sixth embodiment, the incident angle of the measurement light is set between the critical angle $\theta_1$ and the critical angle $\theta_2$ and the high-speed CCD camera 61 detects whether the measurement light is reflected in each area of the surface 26A. The processing unit 62 identifies, based on the result of the detection in the high-speed CCD camera 61, whether the substance that contacts each area of the surface 26A is the tire T or air or water. In this way, the processing unit 62 is able to measure the area in which the tire T contacts the surface 26A. The processing unit 62 is composed of, for example, a computer (in particular, a personal computer), similar to the processing unit 15.

Further, by continuing to measure the contact area of the tire T while rotating the tire T, the processing unit 62 is able to identify a sliding part of the tire T and an adhering part of the tire T in contact with the surface 26A. While the sliding part of the tire T is no longer in contact with the surface 26A for a short period of time, the adhering part of the tire T contacts the surface 26A for a long time, whereby it is possible to differentiate the sliding part from the adhering part by the time during which each of these parts of the tire T contacts the surface 26A.

As described above, by measuring the area in which the tire T contacts the surface 26A, the processing unit 62 is able to measure the relevance between the area in which the tire T contacts the surface 26A and the low-frequency and high-frequency viscoelastic characteristic measurement results. Instead of measuring the time T, another substance that is used for rotation (e.g., sample roller) may be measured. Further, even in a case in which torque is applied in a range of partial sliding without rotating the measurement sample S or a case in which the tire T is simply made contact with the delay member 26, in a way similar to the method stated above, the relevance between the contact area of the measurement sample S and the delay member 26 and the low-frequency and high-frequency viscoelastic characteristic measurement results can be measured.

Furthermore, in the viscoelastic characteristic measurement apparatus 5 shown in Fig. 17, the transducer 25 may not emit the incident sound wave (to measure the high-frequency viscoelasticity) to the tire T and may execute only the reception of the reflected sound wave emitted from the measurement sample S via the delay member 26. The data of the reflected sound wave that has been received is output to the processing unit 15 via the conversion unit 14. The processing unit 15 is able to measure, based on the data of the reflected sound wave, the vibration state of the tire T on the friction surface due to sliding. Accordingly, the viscoelastic characteristic measurement apparatus 5 is able to measure the high-frequency viscoelasticity more appropriately. The viscoelastic characteristic measurement apparatus 5 is able to collect, for example, data of vibration frequency bands, spectra, or waveforms that exhibit significant features. Based on this data, the viscoelastic characteristic measurement apparatus 5 is able to determine whether the vibration state of the tire T is in a desired state when the frictional force is not applied to the measurement sample S. When the vibration state is a desired state, the viscoelastic characteristic measurement apparatus 5 is able to measure the high-frequency viscoelasticity of the measurement sample S, whereby it is possible to make the condition of measuring the high-frequency viscoelasticity optimal. The surface 26A may be a friction surface having a necessary surface roughness in accordance with the measurement.

The present invention is not limited to the aforementioned embodiments and may be changed as appropriate without departing from the spirit of the present invention. For example, in the first embodiment, the temperature detection sensor 33 may not necessarily be provided. Further, in the first embodiment, instead of determining the distortion of the measurement sample S in advance to measure the stress, the stress to be applied to the measurement sample S may be determined in advance to measure the distortion. In this case, a displacement detection sensor is provided in place of the stress detection sensor 32. This displacement detection sensor detects data of distortion of the measurement sample S based on, for example, a displacement of the axis 21 in the vertical direction and outputs the data of the distortion that has been acquired to the processing unit 15. Further, the stress detection sensor 32 and the displacement detection sensor may be both provided in the rheometer unit 11. Further, the device that deforms the measurement sample S is not limited to the rheometer.

The processing shown in the first to sixth embodiments can be executed by a computer as one of control methods. The program can be stored and provided to a computer using any type of non-transitory computer readable media. Non-transitory computer readable media include any type of tangible storage media. Examples of non-transitory computer readable media include magnetic storage media (such as flexible disks, magnetic tapes, hard disk drives, etc.), optical magnetic storage media (e.g., magneto-optical disks), Compact Disc Read Only Memory (CD-ROM), CD-R, CD-R/W, and semiconductor memories (such as mask ROM, Programmable ROM (PROM), Erasable PROM (EPROM), flash ROM, Random Access Memory (RAM), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g., electric wires, and optical fibers) or a wireless communication line

This application is based upon and claims the benefits of priorities from Japanese Patent Application No. 2014-142270, filed on July 10, 2014 and Japanese Patent Application No. 2015-064921, filed on March 26, 2015, the disclosures of which are incorporated herein in their entirety by reference.

**Reference Signs List**

| 1, 2, 3, 4 | VISCOELASTIC CHARACTERISTIC MEASUREMENT APPARATUS |
|---|---|
| 10 | RHEOMETER CONTROLLER |
| 11 | RHEOMETER UNIT |
| 12 | SOUND WAVE EMISSION UNIT |
| 13 | SOUND WAVE SIGNAL GENERATION UNIT |
| 14 | CONVERSION UNIT |
| 15 | PROCESSING UNIT |
| 20 | DRIVE UNIT |
| 21 | AXIS |
| 22 | ANNULAR PLATE |
| 23 | MOTOR |
| 24 | SENSOR |
| 25 | TRANSDUCER |
| 26 | DELAY MEMBER |
| 27 | DRIVE WAVEFORM GENERATOR |
| 28 | DIRECTION REGULATOR |
| 29 | HIGH-FREQUENCY AMPLIFIER |
| 30 | D/A CONVERSION UNIT |
| 31 | A/D CONVERSION UNIT |
| 32 | STRESS DETECTION SENSOR |
| 33 | TEMPERATURE DETECTION SENSOR |

| 34 | INPUT UNIT |
|---|---|
| 35 | OPERATING UNIT |
| 36 | TIME DATA MEMORY UNIT |
| 37 | STORAGE UNIT |
| 38 | DISPLAY/OUTPUT UNIT |
| 39 | REFLECTION MEMBER |
| 40 | TRANSDUCER |
| 41, 42 | ELECTRODE |
| 50, 51 | CASING STRUCTURE |
| 60 | LIGHT SOURCE |
| 61 | HIGH-SPEED CCD CAMERA |
| 62 | PROCESSING UNIT |

**Claims**

1.  A viscoelastic characteristic measurement apparatus comprising:

    a sample deforming unit that applies an external force to a measurement sample to impose a cyclic deformation;
    a first viscoelastic characteristic calculation unit that calculates a low-frequency viscoelastic characteristic of the measurement sample based on stress acting on the measurement sample that has been deformed by the sample deforming unit and distortion of the measurement sample;
    an emission unit that emits an incident sound wave to the measurement sample that has been deformed by the sample deforming unit;
    a receiver that receives a reflected sound wave generated as a result of reflection of the incident sound wave emitted from the emission unit in the measurement sample or a transmitted sound wave generated as a result of transmission of the incident sound wave through the measurement sample; and
    a second viscoelastic characteristic calculation unit that calculates a high-frequency viscoelastic characteristic of the measurement sample at a frequency of the incident sound wave based on the reflected sound wave or the transmitted sound wave received by the receiver.

2.  The viscoelastic characteristic measurement apparatus according to Claim 1, wherein the viscoelastic characteristic measurement apparatus calculates a correlation between the low-frequency viscoelastic characteristic of the measurement sample and the high-frequency viscoelastic characteristic of the measurement sample.

3.  The viscoelastic characteristic measurement apparatus according to Claim 1 or 2, wherein the sample deforming unit rotates the measurement sample about a predetermined rotation axis at a predetermined cycle.

4.  The viscoelastic characteristic measurement apparatus according to any one of Claims 1 to 3, wherein the sample deforming unit vibrates the measurement sample in a predetermined direction at a predetermined cycle.

5.  The viscoelastic characteristic measurement apparatus according to any one of Claims 1 to 4, wherein the sample deforming unit generates stretching or contracting deformation in the measurement sample by applying a tensile force or a compressive force to the measurement sample in a predetermined direction at a predetermined cycle.

6.  The viscoelastic characteristic measurement apparatus according to any one of Claims 1 to 5, wherein the sample deforming unit generates a bending deformation in the measurement sample by applying a bending force to the measurement sample in a predetermined direction at a predetermined cycle.

7.  The viscoelastic characteristic measurement apparatus according to any one of Claims 1 to 6, wherein the viscoelastic characteristic measurement apparatus further comprises an electric field applying unit that applies an electric field to the measurement sample.

8.  The viscoelastic characteristic measurement apparatus according to any one of Claims 1 to 7, further comprising:

    a reflection unit that is connected to the sample deforming unit, transmits the external force to the measurement sample, reflects the transmitted sound wave, and causes the receiver to receive the reflected wave; and
    a casing that encloses the reflection unit and the measurement sample.

9. The viscoelastic characteristic measurement apparatus according to any one of Claims 1 to 7, further comprising:

   a delay unit that is provided between the emission unit and the measurement sample; and
   a reflection unit that reflects the transmitted sound wave and causes the receiver to receive the reflected wave, wherein an acoustic impedance of the delay unit and an acoustic impedance of the reflection unit have substantially the same value.

10. The viscoelastic characteristic measurement apparatus according to any one of Claims 1 to 7, further comprising:

    a delay unit that is provided between the emission unit and the measurement sample and contacts the measurement sample;
    a light source that irradiates a measurement light on a contact surface of the delay unit and the measurement sample;
    a detection unit that detects a reflected light generated as a result of reflection of the measurement light in the contact surface; and
    a calculation unit that calculates an area of the contact surface based on a result of the detection in the detection unit.

11. A viscoelastic characteristic measurement method comprising:

    a deformation step that applies an external force to a measurement sample to impose a cyclic deformation;
    a first calculation step that calculates a low-frequency viscoelastic characteristic of the measurement sample based on stress acting on the measurement sample that has been deformed and distortion of the measurement sample;
    an emission step that emits an incident sound wave to the measurement sample that has been deformed;
    a reception step that receives a reflected sound wave generated as a result of reflection of the incident sound wave that has been emitted in the measurement sample or a transmitted sound wave generated as a result of transmission of the incident sound wave through the measurement sample; and
    a second calculation step that calculates a high-frequency viscoelastic characteristic of the measurement sample at a frequency of the incident sound wave based on the reflected sound wave or the transmitted sound wave that has been received.

Fig. 1

EP 3 168 598 A1

24

32

STRESS DETECTION
SENSOR

33

TEMPERATURE SENSOR

Fig. 2

Fig. 3

- 15
- 34 INPUT UNIT
- 37 STORAGE UNIT
- 38 DISPLAY/OUTPUT UNIT
- 35 OPERATING UNIT
- 36 TIME DATA MEMORY UNIT
- 14
- 30 D/A CONVERSION UNIT
- 31 A/D CONVERSION UNIT

Fig. 4A

AIR

IMPEDANCE $Z_A(f)$

$a_0(f)\exp(i\theta_0(f))$

IMPEDANCE $Z_B(f)$

~26

~25

Fig. 4B

S

IMPEDANCE $Z_S(f)$

$a(f)\exp(i\theta(f))$

IMPEDANCE $Z_B(f)$

~26

~25

```
                    ┌──────────────────┐
                    │      START       │
                    └──────────────────┘
                             │
                             ▼                        S1
              ╱──────────────────────────╲
             ╱        DEFORMATION          ╲
    ┌───────▶         INSTRUCTION           ◀
    │         ╲        RECEIVED?            ╱
    │          ╲──────────────────────────╱
    │    NO              │  Yes
    │                    ▼
    │         ┌──────────────────────────┐      S2
    │         │ DEFORM MEASUREMENT SAMPLE S│
    │         └──────────────────────────┘
    │                    │
    │                    ▼                        S3
    │          ╱──────────────────────────╲
    │         ╱         EMISSION            ╲
    └───────▶         INSTRUCTION            ◀
              ╲         RECEIVED?           ╱
               ╲──────────────────────────╱
         NO              │  Yes
                         ▼
              ┌──────────────────────────┐      S4
              │    EMIT INCIDENT SOUND WAVE │
              └──────────────────────────┘
                         │
                         ▼
              ┌──────────────────────────┐      S5
              │  RECEIVE REFLECTED SOUND WAVE│
              └──────────────────────────┘
                         │
                         ▼
              ┌──────────────────────────┐      S6
              │    ANALYZE WAVEFORM IN     │
              │     FREQUENCY REGION       │
              └──────────────────────────┘
                         │
                         ▼
              ┌──────────────────────────┐      S7
              │      MEASURE STRESS        │
              └──────────────────────────┘
                         │
                         ▼
              ┌──────────────────────────┐      S8
              │       CALCULATE            │
              │ VISCOELASTIC CHARACTERISTIC│
              └──────────────────────────┘
                         │
                         ▼
                        ( A )
```

Fig. 5A

A

EXTRACT FEATURE AMOUNT IN TIME CHANGE — S9

CALCULATE CORRELATION — S10

EXTRACT INFLUENCES OF EXTERNAL FIELD
ON VISCOELASTIC CHARACTERISTIC — S11

Fig. 5B

Fig. 6

2

- 15 PROCESSING UNIT
- 14
- 30 D/A CONVERSION UNIT
- 31 A/D CONVERSION UNIT
- 13
- 27 DRIVE WAVEFORM GENERATOR
- 28 DIRECTION REGULATOR
- 29 HIGH-FREQUENCY AMPLIFIER
- 10 RHEOMETER CONTROLLER
- 20
- 23 MOTOR
- 24 SENSOR
- 26
- 25
- 21
- 22
- 39
- S
- 12
- 11

Fig. 7A

AIR

IMPEDANCE $Z_A$

A0 WAVE
$(a_0(f) \exp(i \theta_0(f)))$

IMPEDANCE $Z_B$

26

25

Fig. 7B

AIR

B1 WAVE

IMPEDANCE $Z_R$

DENSITY $\rho_R$

39

IMPEDANCE $Z_B$

A1 WAVE

26

25

IMPEDANCE $Z_R$      DENSITY $\rho_R$ 39

B WAVE      DENSITY $\rho$    S

IMPEDANCE $Z_S$

IMPEDANCE $Z_B$

A WAVE 26

25

Fig. 7C

Fig. 8A

A

EXTRACT FEATURE AMOUNT IN TIME CHANGE — S9

CALCULATE CORRELATION — S10

EXTRACT INFLUENCES OF EXTERNAL FIELD
ON VISCOELASTIC CHARACTERISTIC — S11

Fig. 8B

Fig. 9

Fig. 10A

Fig. 10B

Fig. 10C

Fig. 11

Fig. 12

Fig. 13A
AIR

IMPEDANCE $Z_A$

$A_0$ WAVE
$(a_0 \cdot \exp(i\theta_0))$

IMPEDANCE $Z_B$

26

25

Fig. 13B

DENSITY $\rho$

S

$R_{BS}$
IMPEDANCE $Z_S$

A WAVE
$(a \cdot \exp(i\theta_A))$

IMPEDANCE $Z_B$

26

25

$R_{BS}$

DENSITY $\rho$

IMPEDANCE $Z_S$

S

39

$T_{BS}$   $T_{SB}$

B WAVE
$(b \cdot \exp(i\theta_B))$

IMPEDANCE $Z_B$

26

25

Fig. 13C

Fig. 14

Fig. 15

Fig. 16

PRESS

ROTATE

26A

T

T1

26

MEASUREMENT
LIGHT

REFLECTED
LIGHT

25

60

61

62

Fig. 17

**INTERNATIONAL SEARCH REPORT**

| International application No. |
|---|
| PCT/JP2015/003431 |

| A. CLASSIFICATION OF SUBJECT MATTER |
|---|
| *G01N19/00(2006.01)i, G01N29/04(2006.01)i* |

According to International Patent Classification (IPC) or to both national classification and IPC

| B. FIELDS SEARCHED |
|---|
| Minimum documentation searched (classification system followed by classification symbols) |
| G01N19/00, G01N29/04 |

Documentation searched other than minimum documentation to the extent that such documents are included in the fields searched

| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| A | JP 2007-121200 A (Omron Corp.), 17 May 2007 (17.05.2007), entire text; all drawings & WO 2007/052601 A1 & EP 1944605 A1 & CN 101300485 A | 1-11 |
| A | JP 2012-47716 A (Ricoh Co., Ltd.), 08 March 2012 (08.03.2012), entire text; all drawings (Family: none) | 1-11 |
| A | JP 1-110255 A (NOK Corp.), 26 April 1989 (26.04.1989), entire text; all drawings (Family: none) | 1-11 |

☐ Further documents are listed in the continuation of Box C.  ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 October 2015 (07.10.15) | 20 October 2015 (20.10.15) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007047130 A
- JP 2004228265 A
- JP 2010048722 A
- JP 2014142270 A
- JP 2015064921 A